(19) Europäisches Patentamt
European Patent Office
Office européen des brevets

(11) Veröffentlichungsnummer: **0 057 160**
**B1**

(12) # EUROPÄISCHE PATENTSCHRIFT

(45) Veröffentlichungstag der Patentschrift: **19.06.85**

(51) Int. Cl.⁴: **C 07 D 249/20**, C 08 K 5/34

(21) Anmeldenummer: **82810020.6**

(22) Anmeldetag: **18.01.82**

(54) **2-(2-Hydroxyphenyl)-benztriazole, ihre Verwendung als UV-Absorber und ihre Herstellung.**

(30) Priorität: **23.01.81 CH 437/81**

(43) Veröffentlichungstag der Anmeldung: **04.08.82 Patentblatt 82/31**

(45) Bekanntmachung des Hinweises auf die Patenterteilung: **19.06.85 Patentblatt 85/25**

(84) Benannte Vertragsstaaten: **BE DE FR GB IT NL**

(56) Entgegenhaltungen:
**US - A - 3 213 058**
**US - A - 3 766 205**
**US - A - 4 219 480**
**US - A - 4 230 867**

**Chemical Abstracts Band 84, Nr. 22 31. Mai 1976 Columbus, Ohio, USA K. KOGA et al. "Hydroxybenzotriazoles as ultraviolet light-absorbing agents" Seite 347, Spalte 1, Abstract Nr. 155517g**

(73) Patentinhaber: **CIBA-GEIGY AG, Postfach, CH-4002 Basel (CH)**

(72) Erfinder: **Rody, Jean, Dr., Rütiring 82, CH-4125 Riehen (CH)**
Erfinder: **Slongo, Mario, Dr., Pappelweg 8, CH-4132 Muttenz (CH)**

**EP 0 057 160 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue 2-(2-Hydroxyphenyl)-benztriazole, welche als UV-Stabilisatoren für organisches Material, insbesondere in Kunststoffen und in photographischem Material, Verwendung finden.

Es sind bereits Lichtschutzmittel für Kunststoffe auf Benzotriazol-Basis bekannt, welche am aromatischen Substituenten in 2-Stellung des Benztriazol-Kernes lediglich eine OH-Gruppe und eine verzweigte Alkylgruppe, sonst aber keine funktionelle Gruppe enthalten. Beispiele für solche UV-Absorber sind das 2-(2-Hydroxy-3,5-dicumyl-phenyl)-benztriazol und das 2-(2-Hydroxy-5-tert.-butyl-phenyl)-benztriazol. Diese bekannten UV-Stabilisatoren sind zum Teil nur durch aufwendige Verfahren herstellbar.

Außerdem sind sie größtenteils schwer löslich und relativ flüchtig. Sie neigen stark zur Kristallisation und Migration. Diese für die UV-Stabilisierung sehr ungünstigen Eigenschaften stören stark bei der Verwendung in Kunststoffen bzw. Lacken, wie z. B. Automobillacken.

In der US-PS 3 766 205 sind Lichtstabilisatoren der Formel

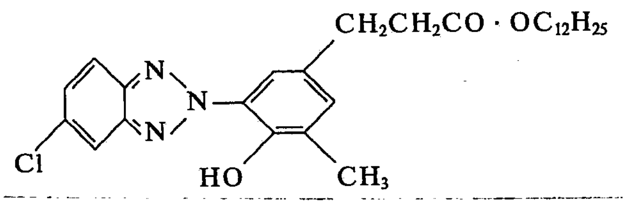

beschrieben, welche beispielsweise in Polyamiden und Polyestern eingesetzt werden. Sie weisen dieselben Nachteile wie die oben beschriebenen UV-Stabilisatoren auf. In der JP-Patentanmeldung Sho 54-95 233 werden ähnliche Lichtstabilisatoren z. B. der Formel

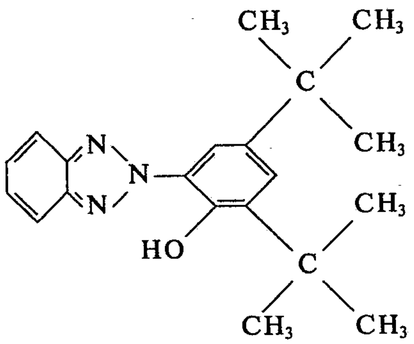

beschrieben, welche zusammen mit Co-Stabilisatoren in Materialien für die Farbphotographie eingesetzt werden. Diese Stabilisatoren sind in ihre Wirkung wie die oben beschriebenen Lichtstabilisatoren nicht optimal.

Der Schutz gegen UV-Schäden ist bei der Phototechnik und insbesondere in der Farbphototechnik von besonderer Bedeutung.

Um die in einem farbphotographischen Material vorhandenen Komponenten (insbesondere Farbstoffe und Kuppler) möglichst wirkungsvoll gegen die Zerstörung durch ultraviolettes Licht zu schützen, werden normalerweise UV-Absorber in eine oder mehrere der oberen Schichten des Materials eingelagert. Hierzu wird der UV-Absorber in der Regel in einem hochsiedenden Lösungsmittel gelöst und diese Lösung in Form sehr feiner Tröpfchen in der betreffenden Gießlösung dispergiert. Da diese Tröpfchen die mechanischen Eigenschaften der Schicht ungünstig beeinflussen und, wenn sie sich in der obersten Schicht des Materials befinden, »ausschwitzen« können, ist es wichtig, die Menge an Absorber-Lösung möglichst gering zu halten. Dies gestattet auch die Herstellung dünnerer Schichten, was wiederum bei der Verarbeitung (Badverschleppung und Trocknung) Vorteile bietet. Man ist deshalb bestrebt, UV-Absorber mit einer möglichst guten Löslichkeit in den üblichen hochsiedenden Lösungsmitteln einzusetzen. Die UV-Absorber des Standes der Technik, wie beispielsweise die in der JP-Anmeldung Sho 54-95 233 offenbarten Stabilisatoren, erfüllen auch diese Anforderung nicht in befriedigendem Maße.

Es wurde nun gefunden, daß die Produkte der vorliegenden Erfindung eine außerordentlich gute Löslichkeit in den hochsiedenden Lösungsmitteln besitzen und deshalb den Produkten des Standes der Technik auch auf dem Photogebiet überlegen sind. In einigen Fällen sind sie sogar flüssig und können ohne hochsiedende Lösungsmittel in einem farbphotographischen Material eingesetzt werden. Im übrigen sind sie auch schwer flüchtig und neigen nicht zur Kristallisation.

Gegenstand der Erfindung sind Verbindungen der Formel I

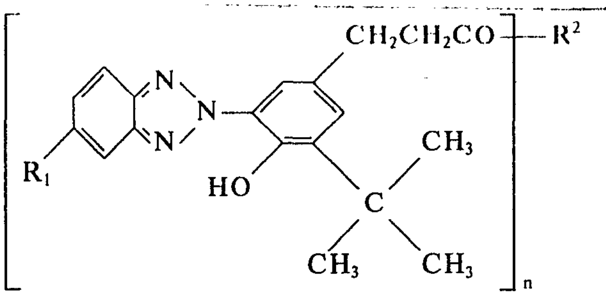

(I)

in der $R^1$ H, Cl, geradkettiges oder verzweigtes $C_1-C_4$ Alkyl oder geradkettiges oder verzweigtes $C_1-C_4$ Alkoxy und n eine der Zahlen 1 oder 2 bedeuten und in der $R^2$

a) im Falle von n = 1

Cl $-OR^3$ oder 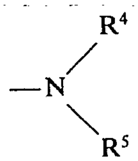

und

b) im Falle von n = 2 einen der zweiwertigen Reste

$-O-R^9-O-$ oder 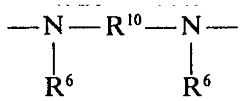

darstellt, wobei

$R^3$ H, gegebenenfalls durch 1 bis 3 OH-Gruppen oder durch

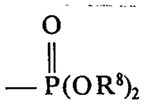 $-O-Si(R^6)_3$ oder $-O-CO(R^6)$

substituiertes geradkettiges oder verzweigtes $C_1-C_{18}$ Alkyl, durch $-O-$, $-S-$ oder $-NR^6-$ ein- oder mehrfach unterbrochenes, geradkettiges oder verzweigtes $C_3-C_{18}$-Alkyl, das gegebenenfalls durch OH oder durch $-O-CO(R^6)$ substituiert sein kann, gegebenenfalls durch OH substituiertes $C_5-C_{12}$-Cycloalkyl, gegebenenfalls durch OH substituiertes, geradkettiges oder verzweigtes $C_2-C_{18}$-Alkenyl, $C_7-C_{15}$-Aralkyl,

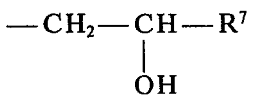 oder 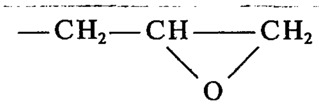

und

$R^4$ und $R^5$ unabhängig voneinander H, geradkettiges oder verzweigtes $C_1-C_{18}$-Alkyl, durch O, S oder $-NR^6-$ ein- oder mehrfach unterbrochenes, geradkettiges oder verzweigtes $C_3-C_{18}$-Alkyl, $C_5-C_{12}$-Cycloalkyl, $C_6-C_{14}$-Aryl, geradkettiges oder verzweigtes $C_3-C_8$-Alkenyl, $C_7-C_{15}$-Aralkyl, $C_6-C_{14}$-Aryl oder $C_1-C_3$-Hydroxyalkyl bedeuten, oder aber $R^4$ und $R^5$ zusammen mit dem Sticksstoffatom einen Pyrrolidin-, Piperidin-, Piperazin- oder Morpholinring darstellen, und wobei

$R^6$ H, geradkettiges oder verzweigtes $C_1-C_{18}$-Alkyl, $C_5-C_{12}$-Cycloalkyl, geradkettiges oder verzweigtes $C_3-C_8$-Alkenyl, $C_6-C_{14}$-Aryl oder $C_7-C_{18}$-Aralkyl,

$R^7$ H, geradkettiges oder verzweigtes, gegebenenfalls durch $-PO(OR^8)_2$ substituiertes $C_1-C_{18}$-Alkyl, gegebenenfalls durch OH substituiertes Phenyl, $C_7-C_{18}$-Aralkyl, oder $-CH_2OR^8$,

$R^8$ geradkettiges oder verzweigtes $C_1-C_{18}$-Alkyl, geradkettiges oder verzweigtes $C_3-C_{18}$-Alkenyl, $C_5-C_{10}$-Cycloalkyl, $C_6-C_{16}$-Aryl oder $C_7-C_{15}$-Aralkyl,

$R^9$ $C_2-C_8$-Alkylen, $C_4-C_8$-Alkenylen, $C_4$-Alkinylen, Cyclohexylen, durch $-O-$ ein- oder mehrfach unterbrochenes, geradkettiges oder verzweigtes $C_4-C_{10}$-Alkylen,

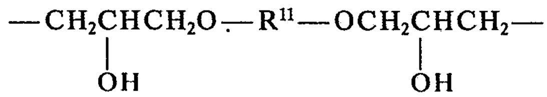 oder 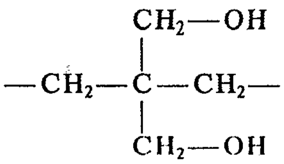

und

$R^{10}$ geradkettiges oder verzweigtes, gegebenenfalls durch —O— ein- oder mehrfach unterbrochenes $C_2$—$C_{12}$-Alkylen, Cyclohexylen,

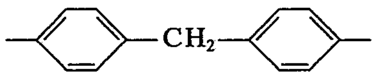 oder 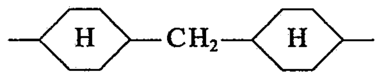

bedeuten, oder aber

$R^{10}$ und $R^6$ zusammen mit den beiden Stickstoffatomen einen Piperazinring darstellen, und wobei

$R^{11}$ geradkettiges oder verzweigtes $C_2$—$C_8$-Alkylen, durch —O— ein- oder mehrfach unterbrochenes, geradkettiges oder verzweigtes $C_4$—$C_{10}$-Alkylen, 1,3- oder 1,4-Cyclohexylen, 1,3- oder 1,4-Phenylen,

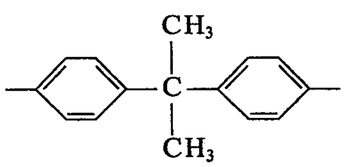 oder 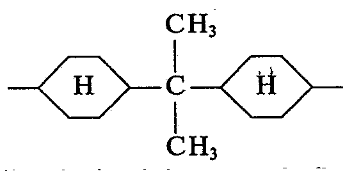

bedeutet.

$R^1$ kann als Alkylrest z. B. Methyl, Äthyl, Propyl, i-Propyl, Butyl und tert.-Butyl, als Alkoxyrest z. B. Methoxy, Äthoxy, Propoxy und Butoxy bedeuten.

$R^3$, $R^4$, $R^5$, $R^6$, $R^7$ und $R^8$ können z. B. folgende Alkylreste bedeuten: Methyl, iso-Propyl, n-Butyl, sec.-Butyl, tert.-Butyl, tert.-Amyl, 2-Äthylhexyl, n-Octyl, 1,1,3,3-Tetramethylbutyl, n-Dodecyl, 1,1,7,7-Tetramethyloctyl, n-Octadecyl.

$R^3$, $R^4$ und $R^5$ können z. B. folgende $C_3$—$C_{18}$-Alkylreste, welche durch O, S oder —$NR^6$— unterbrochen sind, und durch OH substituiert sein können, bedeuten:

Methoxyäthyl, Äthoxyäthyl, Butoxyäthyl, Butoxypropyl, Methylthiaäthyl,

$CH_3OCH_2CH_2OCH_2CH_2$— $CH_3CH_2OCH_2CH_2OCH_2CH_2$—

$C_4H_9OCH_2CH_2OCH_2CH_2$—

Äthylthiapropyl, Octylthiapropyl, Dodecyloxypropyl, 2-Hydroxyäthyl, 2-Hydroxypropyl, 4-Hydroxybutyl, 6-Hydroxyhexyl,

—$CH_2CH_2$—NH—$C_4H_9$ —$CH_2CH_2CH_2NH$—$C_8H_{17}$

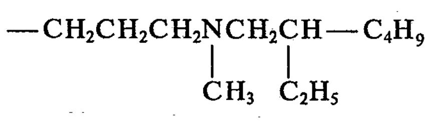

$R^3$, $R^4$, $R^5$, $R^6$ und $R^8$ können z. B. folgende $C_5$—$C_{12}$-Cycloalkylreste bedeuten: Cyclopentyl, Cyclohexyl, Cycloheptyl, Cyclooctyl oder Cyclodecyl. Im Falle von $R^3$ kann der Rest auch OH-substituiert sein.

$R^4$, $R^5$ und $R^6$ können z. B. folgende Alkenylreste bedeuten: Allyl, Methallyl, 2-n-Hexenyl, 4-n-Octenyl.

Wenn $R^3$ Alkenyl bedeutet, so kann es dieselbe Bedeutung wie $R^4$, $R^5$ und $R^6$ als Alkenylreste haben, es kann aber auch z. B. $-CH=CH_2$, 10-n-Undecenyl oder 9-n-Octadecenyl darstellen, wobei der Rest $R^3$ auch OH-substituiert sein kann.

$R^4$ und $R^5$ können z. B. folgende $C_7$—$C_{15}$ Aralkylreste bedeuten: Benzyl, $\alpha$-Phenyläthyl, 2-Phenyläthyl, 4-tert.-Butylbenzyl.

Wenn $R^3$, $R^6$, $R^7$ oder $R^8$ Aralkyl bedeuten, so können sie unabhängig voneinander dieselbe Bedeutung wie $R^4$ bzw. $R^5$ haben.

$R^4$, $R^5$ und $R^6$ können unabhängig voneinander z. B. folgende $C_6$—$C_{14}$-Arylreste bedeuten: Phenyl, $\alpha$-Naphthyl, $\beta$-Naphthyl.

Bedeuten $R^4$ und $R^5$ $C_1-C_3$-Hydroxyalkyl, so kann es sich um folgende Reste handeln: Hydroxymethyl, Hydroxyäthyl, Hydroxypropyl.

$R^9$ und $R^{11}$ können als $C_2-C_8$-Alkylen z. B. folgende Reste bedeuten: Äthylen, Propylen, Butylen, Hexylen, Octylen.

$R^{10}$ kann als Alkylen dieselben Reste bedeuten aber zusätzlich auch noch höhermolekulare Gruppen, wie Decylen, Dodecylen darstellen.

Ist $R^9$ ein $C_4-C_8$-Alkenylen, so kommt z. B. folgende Gruppe in Betracht: Butenylen.

Als durch $-O-$ unterbrochene, geradkettige oder verzweigte $C_4-C_{10}$-Alkylengruppen kommen im Falle $R^9$ und $R^{11}$ z. B. die folgenden Gruppen in Frage:

$-CH_2CH_2OCH_2CH_2-$ 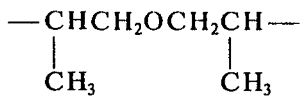 $-CH_2CH_2OCH_2CH_2OCH_2CH_2-$

$-CH_2CH_2OCH_2CH_2OCH_2CH_2OCH_2CH_2-$

Eine Vorzugsform der Erfindung stellen solche Verbindungen der Formel I dar, in der $R^1$ H oder Cl, n die Zahl 1 und $R^2$

$-OR^3$ oder 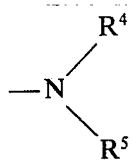

bedeuten, wobei

$R^3$ geradkettiges oder verzweigtes $C_4-C_{12}$-Alkyl, durch $-O-$ unterbrochenes, geradkettiges oder verzweigtes $C_3-C_{10}$-Alkyl, Cyclohexyl oder

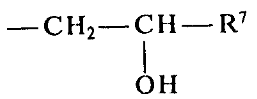

$R^4$ und $R^5$ unabhängig voneinander geradkettiges oder verzweigtes $C_4-C_{12}$-Alkyl, durch $-O-$ unterbrochenes, geradkettiges oder verzweigtes $C_3-C_{10}$-Alkyl oder Cyclohexyl,

$R^7$ geradkettiges oder verzweigtes, gegebenenfalls durch

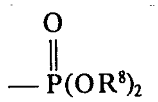

substituiertes $C_1-C_{12}$-Alkyl, oder $-CH_2OR^8$ und

$R^8$ geradkettiges oder verzweigtes $C_1-C_{12}$-Alkyl oder Cyclohexyl darstellen.

Eine weitere Vorzugsform der Erfindung stellen solche Verbindungen der Formel I dar, in der $R^1$ H oder Cl, n die Zahl 2, und $R^2$ einen der zweiwertigen Reste

$-O-R^9-O-$ oder 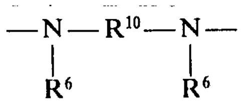

bedeuten, wobei

$R^6$ H oder $C_1-C_8$-Alkyl,

$R^9$ $C_2-C_6$-Alkylen, durch $-O-$ unterbrochenes, geradkettiges oder verzweigtes $C_4-C_{10}$-Alkylen oder

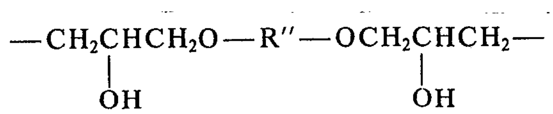

$R^{10}$ geradkettiges oder verzweigtes $C_2-C_{12}$-Alkylen, und

$R^{11}$ $C_2-C_6$-Alkylen oder durch $-O-$ unterbrochenes, geradkettiges oder verzweigtes $C_4-C_{10}$-Alkylen bedeuten.

Typische Vertreter von Verbindungen der Formel I, in der n die Zahl 1 bedeutet, sind die folgenden:

2-[2-Hydroxy-3-tert.-butyl-5-(2-carboxyäthyl)-phenyl]-benztriazol,
2-[2-Hydroxy-3-tert.-butyl-5-(2-carboxyäthyl)-phenyl]-5-chlorbenztriazol,
2-[2-Hydroxy-3-tert.-butyl-5-(2-chlorocarbonyläthyl)-phenyl]-benztriazol,
2-[2-Hydroxy-3-tert.-butyl-5-(2-chlorocarbonyläthyl)-phenyl]-5-chlorbenztriazol,
2-[2-Hydroxy-3-tert.-butyl-5-(2-carbomethoxyäthyl)-phenyl]-benztriazol,
2-[2-Hydroxy-3-tert.-butyl-5-(2-carbomethoxyäthyl)-phenyl]-5-chlorbenztriazol,
2-[2-Hydroxy-3-tert.-butyl-5-(2-carbocyclohexyloxyäthyl)-phenyl]-benztriazol,
2-[2-Hydroxy-3-tert.-butyl-5-(2-carbooctyloxyäthyl)-phenyl]-benztriazol,
2-{2-Hydroxy-3-tert.-butyl-5-[2-carbo-(2-äthylhexyloxy)-äthyl]-phenyl}-benztriazol,
2-[2-Hydroxy-3-tert.-butyl-5-(2-carbo-iso-decyloxyäthyl)-phenyl]-benztriazol,
2-[2-Hydroxy-3-tert.-butyl-5-(2-carbododecyloxyäthyl)-phenyl]-benztriazol,
2-[2-Hydroxy-3-tert.-butyl-5-(2-carbododecyloxyäthyl)-phenyl]-5-chlorbenztriazol,
2-[2-Hydroxy-3-tert.-butyl-5-(2-carbooctyloxyäthyl)-phenyl]-5-chlorbenztriazol,
2-{2-Hydroxy-3-tert.-butyl-5-[2-carbo-(2-äthylhexyloxy)-äthyl]-phenyl}-5-chlorbenztriazol,
2-{2-Hydroxy-3-tert.-butyl-5-[2-carbo-(2-hydroxycyclohexyloxy)-äthyl]-phenyl}-benztriazol,
2-[2-Hydroxy-3-tert.-butyl-5-(2-carbopiperidylamidoäthyl)-phenyl)-benztriazol,
2-[2-Hydroxy-3-tert.-butyl-5-(2-carbomorpholinamidoäthyl)-phenyl]-benztriazol,
2-{2-Hydroxyl-3-tert.-butyl-5-[2-carbo-(3,5-di-tert.-butyl-4-hydroxyanilido)-äthyl]-phenyl}-benztriazol.

Typische Vertreter von Verbindungen der Formel I, in der n dieZahl 2 bedeutet, sind die folgenden:

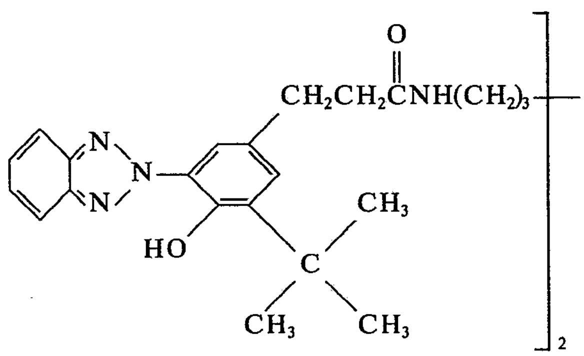

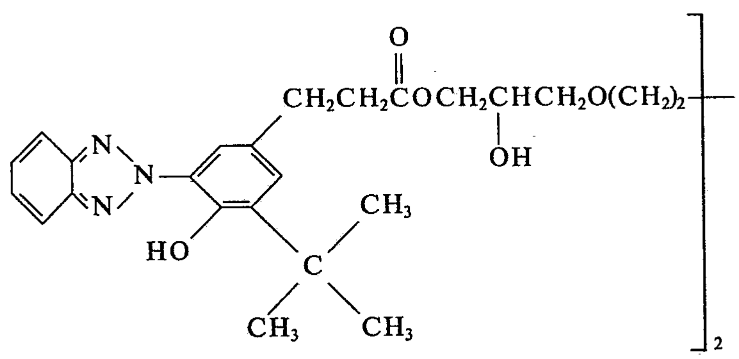

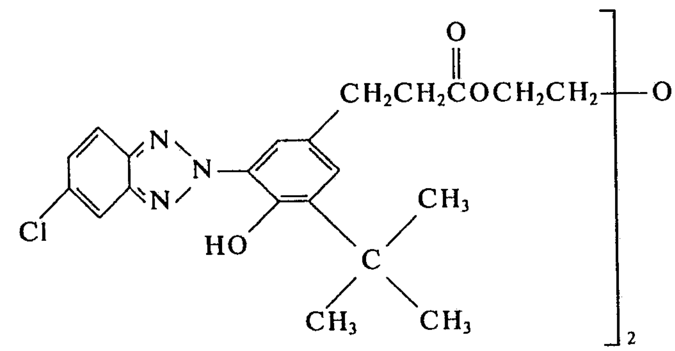

Weitere Vorzugsformen der Erfindung sind folgende Verbindungen:

2-[2-Hydroxy-3-tert.butyl-5-(2-carbo-n-octyloxyäthyl)-phenyl]-benztriazol,
2-{2-Hydroxy-3-tert.butyl-5-[2-carbo-(2-äthylhexyl)-oxyäthyl]-phenyl}-benztriazol,
2-[2-Hydroxy-3-tert.butyl-5-(2-carbo-n-octyloxyläthyl)-phenyl]-5-chlorbenztriazol,
2-{2-Hydroxy-3-tert.butyl-5-[2-carbo-(2-äthylhexyl)-oxyäthyl]-phenyl}-5-chlorbenztriazol und die Verbindung der Formel

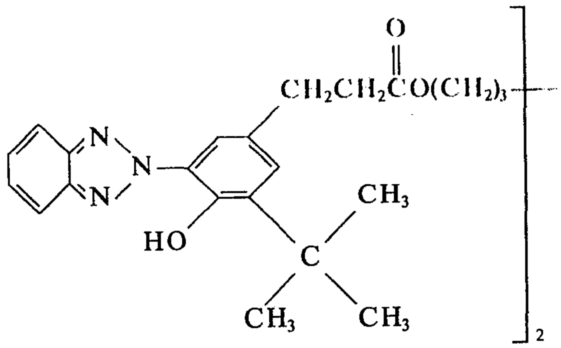

Eine weitere Vorzugsform der Erfindung stellt auch ein Gemisch von Verbindungen der Formel I dar, wobei
2-[2-Hydroxy-3-tert.butyl-5-(2-carbo-n-octyloxyäthyl)-phenyl]-5-chlorbenztriazol und
2-{2-Hydroxy-3-tert.butyl-5-[2-carbo-(2-äthylhexyl)-oxyäthyl]-phenyl}-5-chlorbenztriazol
im Gewichtsverhältnis 1 : 1 vorliegen.

Bei der an sich bekannten Herstellung der erfindungsgemäßen Verbindungen der Formel I wird in folgender Weise verfahren. Ein gegebenenfalls substituiertes o-Nitranilin wird diazotiert und mit 2-(3-tert.butyl-4-hydroxyphenyl)-propionsäure bei pH=8 bis 11 gekuppelt. Der Farbstoff wird nach Ansäuern isoliert. Die Reaktion verläuft nach folgender Formelgleichung

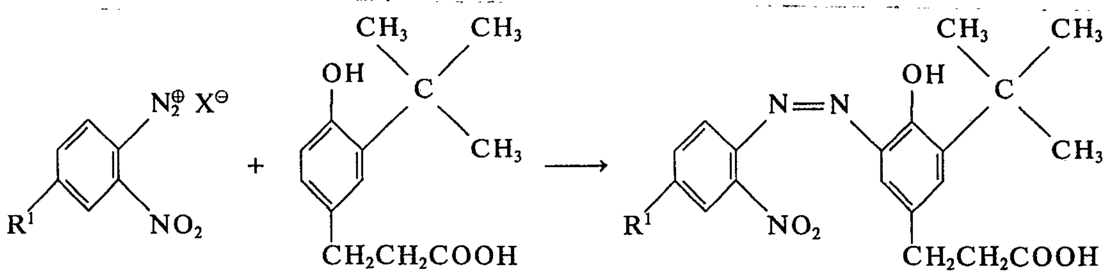

($X^{\ominus}$ bedeutet z. B. ein Halogen-anion (wie $Cl^{\ominus}$), $—HSO_4^{\ominus}$ oder $(BF_4)^{\ominus}$.)

Aus dem erhaltenen Farbstoff wird durch reduzierende Triazolierung unter alkalischen Bedingungen die entsprechende Benztriazolcarbonsäure hergestellt, welche selbst eine erfindungsgemäße Substanz darstellt, welche aber auch als Ausgangssubstanz für andere Verbindungen der Formel I verwendet werden kann. Bei der reduzierenden Triazolierung erfolgt die Reduktion vorzugsweise katalytisch durch Wasserstoff oder es wird Zinkstaub als Reduktionsmittel verwendet. Die Reaktion läuft nach folgendem Schema ab:

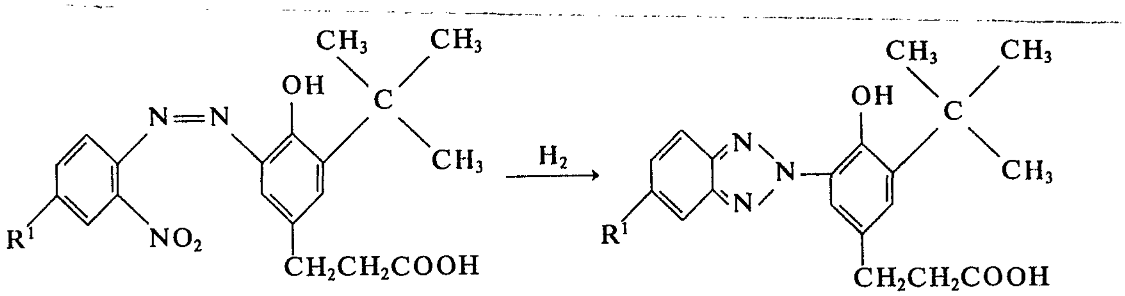


Werden die nach obigem Verfahren erhältlichen Benztriazolcarbonsäuren weiter als Ausgangsstoffe für die Herstellung von anderen durch die Erfindung umfaßten Verbindungen verwendet, so wird nach folgenden Herstellungsverfahren vorgegangen:

1) Direkte Veresterung dieser Carbonsäuren im Falle von n=1 mit Alkoholen der Formel $R^{3'}$ OH ($R^{3'}$ hat dieselbe Definition, wie der vorher angegebene Rest $R^3$, bedeutet aber nicht

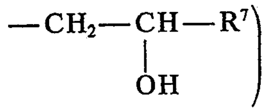

am Wasserabscheider in Gegenwart eines sauren Katalysators; im Falle von n=2 entsprechende direkte Veresterung mit Dialkoholen der Formel HO—$R^9$—OH.

2) Umsetzung der Benztriazolcarbonsäure mit einem Epoxid nach folgendem Formelschema

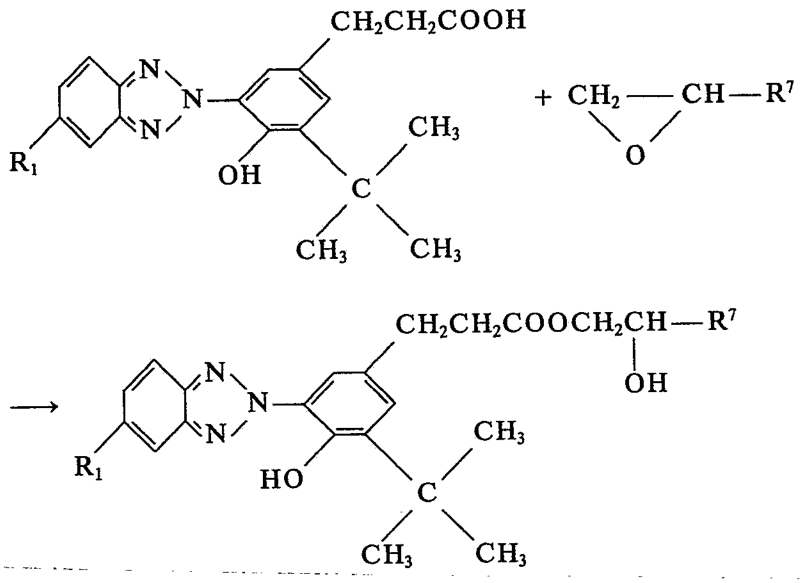

Ein weiterer Einbau in Lackbindemittel ist möglich. Der Einbau ist sowohl bei der Harzherstellung als auch bei der chemischen Vernetzung von Lackharzen während des Einbrennvorgangs denkbar.

Bei der Harzsynthese erfolgt der Einbau z. B. durch Polykondensation (Polyesterharze, Alkydharze) oder durch Polymerisation (Acrylharze) wenn $R_7$ eine Vinylgruppe enthält. Beim Einbrennvorgang erfolgt der Einbau durch Reaktion der Alkoholgruppe mit Vernetzern wie z. B. Amino-/Formaldehydharzen oder Polyisocyanaten.

3) Chlorierung der Benztriazolcarbonsäure nach bekannten Verfahren zum erfindungsgemäßen Säurechlorid der Formel I. Man suspendiert die Ausgangssäure in z. B. Hexan, gibt bei Raumtemperatur tropfenweise Thionylchlorid (50%iger Überschuß) zu. Unter Exothermie gehen HCl und $SO_2$ gasförmig ab und es ensteht eine Lösung. Das erhaltene Säurechlorid wird bei 60° C ausgerührt.

Die nach der Reaktion 1. erhältlichen erfindungsgemäßen Benztriazolalkylester (insbesondere diejenigen mit niedermolekularen Alkylgruppen, wie $-CH_3$ und $-C_2H_5$), welche auch noch nach anderen bekannten Esterherstellungsverfahren herstellbar sind, können ebenfalls als Ausgangssubstanzen für die Herstellung weiterer erfindungsgemäßer Verbindungen der Formel I eingesetzt werden. Sie werden beispielsweise bei folgenden Verfahren verwendet.

4) Umesterung der erfindungsgemäßen Benztriazolcarbonsäuremethylester im Falle von n=1 in Formel I mit Alkoholen der Formel $R^{3'}$ OH in Gegenwart eines Umesterungskatalysators, wie Tetrabutylorthotitanat, Li-Amid und Na-Methylat, unter Abdestillieren des gebildeten Methanols. Es entstehen so erfindungsgemäße, höhermolekulare Benztriazolsäureester.

Eine entsprechende Umesterung ist auch im Falle von n=2 in Formel I mit Dialkoholen der Formel HO—$R^9$—OH möglich, wobei die Menge an diesem Dialkohol entsprechend der Reaktionsgleichung auf die Hälfte herabgesetzt wird.

Bei der Herstellung von Verbindungen der Formel I, welche eine oder mehrere Amidgruppen enthalten, geht man folgendermaßen vor.

5) Thermische Umsetzung von Benztriazolcarbonsäureestern (bevorzugt Methyl- oder Äthylester), gegebenenfalls in Gegenwart von Katalysatoren, wie z. B. Li-Amid oder Na-Methylat, mit Ammoniak oder einem primären oder einem sekundären Amin der Formel

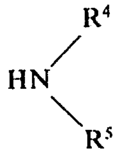

oder aber im Falle von n = 2 in Formel I mit Diaminen der Formel

$$\begin{array}{ccc} HN & —R^{10}— & NH \\ | & & | \\ R^6 & & R^6 \end{array}$$

jeweils unter Abdestillation des freiwerdenden Alkohols zu den entsprechenden Benztriazolcarbonsäureamiden.

6) Dieselben Amide kann man aber auch durch Umsetzung der beispielsweise nach Reaktion 3 erhältlichen erfindungsgemäßen Säurechloride der Formel I mit Ammoniak oder einem primären oder sekundären Mono- oder Diamin in Gegenwart eines Säureakzeptors (wie z. B. Triäthylamin) erhalten.

7) Einbau der Benztriazolcarbonsäure in Lackbindemittel wie z. B. PES-Harze oder Epoxidharze.

Die als Ausgangssubstanzen für die Herstellung der erfindungsgemäßen Benztriazolcarbonsäuren verwendeten o-Nitraniline und die 2-[3-tert.Butyl-4-hydroxyphenyl)-propionsäure sind bekannte Verbindungen, welche auch nach bekannten Verfahren hergestellt werden können. Dagegen sind die als Zwischenprodukte anfallenden Diazofarbstoffe neue Substanzen.

Die erfindungsgemäßen Verbindungen sind wirksame Lichtstabilisatoren für organische Materialien, so z. B. für eine große Anzahl Polymere. Für Applikationen in farbphotographischem Material oder in Lacken und überall, wo die Anwendung flüssiger Lichtschutzmittel entscheidende Vorteile bringt, ist der Einsatz von Gemischen von Produkten der vorliegenden Erfindung, wie z. B. Gemische bestehend aus 2 oder mehreren Estern, ganz besonders vorteilhaft. Bevorzugt werden die erfindungsgemäßen Stabilisatoren oder deren Gemische in einer Menge von 0,1—5 Gew.-%, insbesondere 0,5 bis 3 Gew.-%, bezogen auf das organische Material, eingesetzt. Geeignete Polymere sind:

1. Polymere von Mono- und Diolefinen, beispielsweise Polyäthylen (das gegebenenfalls vernetzt sein kann), Polypropylen, Polyisobutylen, Polybuten-1, Polymethylpenten-1, Polyisopren oder Polybutadien sowie Polymerisate von Cycloolefinen wie z. B. von Cyclopenten oder Norbornen.
2. Mischungen der unter 1) genannten Polymeren, z. B. Mischungen von Polypropylen mit Polyäthylen oder mit Polyisobutylen.
3. Copolymere von Mono- und Diolefinen untereinander oder mit anderen Vinylmonomeren, wie z. B. Äthylen-Propylen-Copolymere, Propylen-Buten-1-Copolymere, Propylen-Isobutylen-Copolymere, Äthylen-Buten-1-Copolymere, Propylen-Butadien-Copolymere, Isobutylen-Isopren-Copolymere, Äthylen-Äthylacrylat-Copolymere, Äthylen-Alkylmethacrylat-Copolymere, Äthylen-Vinylacetat-Copolymere oder Äthylen-Acrylsäure-Copolymere und deren Salze (Ionomere), sowie Terpolymere von Äthylen mit Propylen und einem Dien, wie Hexadien, Dicyclopentadien oder Äthylidennorbornen.
4. Polystyrol.
5. Statistische Copolymere von Styrol oder $\alpha$-Methylstyrol mit Dienen oder Acrylderivaten, wie z. B. Styrol-Butadien, Styrol-Acrylnitril, Styrol-Äthylmethacrylat, Styrol-Acrylnitril-Methacrylat; Mischungen von hoher Schlagzähigkeit aus Styrol-Copolymeren und einem anderen Polymer wie z. B. einem Polyacrylat, einem Dien-Polymeren oder einem Äthylen-Propylen-Dien-Terpolymeren; sowie Block-Copolymere des Styrols, wie z. B. Styrol-Butadien-Styrol, Styrol-Ispren-Styrol, Styrol-Äthylen/Butylen-Styrol oder Styrol-Äthylen/Propylen-Styrol.
6. Pfropfcopolymere von Styrol, wie z. B. Styrol auf Polybutadien, Styrol und Acrylnitril auf Polybutadien, Styrol und Alkylacrylate, bzw. Alkylmethacrylate auf Polybutadien, Styrol und Acrylnitril auf Äthylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z. B. als sogenannte ABS, MBS, ASA oder AES-Polymere bekannt sind.
7. Halogenhaltige Polymere, wie z. B. Polychloropren, Chlorkautschuk, chloriertes oder chlorsulfoniertes Polyäthylen, insbesondere Polymere aus halogenhaltigen Vinylverbindungen, wie z. B. Polyvinylchlorid, Polyvinylidenchlorid, Polyvinylfluorid, Polyvinylidenfluorid; sowie deren Copolymeren wie Vinylchlorid-Vinylidenchlorid, Vinylchlorid-Vinylacetat oder Vinylidenchlorid-Vinylacetat.
8. Polymere, die sich von $\alpha,\beta$-ungesättigten Säuren und deren Derivaten ableiten, wie Polyacrylate und Polymethacrylate, Polyacrylamide und Polyacrylnitrile.
9. Copolymere der unter 8) genannten Monomeren untereinander oder mit anderen ungesättigten Monomeren, wie z. B. Acrylnitril-Butadien-Copolymere, Acrylnitril-Alkylacrylat-Copolymere, Acrylnitril-Vinylchlorid-Copolymere, oder Acrylnitril-Alkylmethacrylat-Butadien-Terpolymere.
10. Polymere, die sich von ungesättigten Alkoholen und Aminen bzw. deren Acylderivaten oder Acetalen ableiten, wie Polyvinylakohol, Polyvinylacetat-, -stearat, -benzoat, -maleat, Polyvinylbutyral, Polyallylphthalat, Polyallylmelamin.
11. Homo- und Copolymere von cyclischen Äthern, wie Polyalkylenglykole, Polyäthylenoxyd, Polypropylenoxyd oder deren Copolymere mit Bisglycidyläthern.

12. Polyacetale, wie Polyoxymethylen, sowie solche Polyoxymethylene, die Comonomere wie z. B. Äthylenoxyd enthalten.
13. Polyphenylenoxyde und -sulfide, sowie Mischungen von Polyphenylenoxyden und Polystyrol.
14. Polyurethane, die sich von Polyäthern, Polyestern und Polybutadienen mi endständigen Hydroxylgruppen einerseits und aliphatischen oder aromatischen Polyisocyanaten andererseits ableiten sowie deren Vorprodukte.
15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, Polyamid 6/10, Polyamid 11, Polyamid 12, Poly-2,4,4-trimethylhexamethylen-terephthalamid, Poly-m-phenylen-isophthalamid, sowie deren Copolymere mit Polyäthern wie z. B. mit Polyäthylenglykol, Polypropylenglykol oder Polytetramethylenglykol.
16. Polyharnstoffe, Polyimide und Polyamid-imide.
17. Polyester, die sich von Dicarbonsäuren und Dialkoholen und/oder von Hydroxycarbonsäuren oder den entsprechenden Lactonen ableiten, wie Polyäthylenterephthalat, Polybutylenterephthalat, Poly-1,4-dimethylolcyclohexanterephthalat, Polyhydroxybenzoate, sowie Block-Polyäther-ester, die sich von Polyäthern mit Hydroxyendgruppen ableiten.
18. Polycarbonate.
19. Polysulfone und Polyäthersulfone.
20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.
21. Trocknende und nicht-trocknende Alkydharze.
22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.
23. Vernetzbare Acrylharze, die sich von substituierten Acrylsäureestern ableiten wie z. B. von Epoxyacrylaten, Urethan-acrylaten oder Polyester-acrylaten.
24. Alkydharze, Polyesterharze und Acrylatharze, die mit Melaminharzen, Harnstoffharzen, Polyisocyanaten oder Epoxidharzen vernetzt sind.
25. Vernetzte Epoxidharze, die sich von Polyepoxiden ableiten, z. B. von Bis-glycidyläthern oder von cycloaliphatischen Diepoxiden.
26. Natürliche Polymere, wie Cellulose, Naturkautschuk, Gelatine, sowie deren polymerhomolog chemisch abgewandelte Derivte, wie Celluloseacetate, -propionate und -butyrate, bzw. die Celluloseäther, wie Methylcellulose.

Gegenstand der Erfindung sind somit auch stabilisierte, organische Materialien, insbesondere synthetische Polymere, welche vorzugsweise 0,1 bis 5 Gew.-% einer Verbindung der Formel I in der Masse enthalten.

Die erfindungsgemäßen Verbindungen sind nicht nur wirksame Lichtschutzmittel, wie die bekannten UV-Absorber auf Benztriazol-Basis, sondern dank ihres überraschend geringen Verlustes durch Verflüchtigung bei hoher Temperatur auch besonders wertvoll zum Stabilisieren von Polymeren, welche bei hohen Temperaturen verarbeitet werden müssen.

Die erfindungsgemäßen Verbindungen werden daher vorzugsweise zum Stabilisieren von Polyestern wie z. B. Polyäthylenterephthalat, Polybutylenterephthalat oder deren Copolymeren, von Polycarbonaten, z. B. aus Bisphenol A und Phosgen oder deren Copolymeren, von Polyamiden, z. B. Nylon--6, Nylon-6,6, Nylon-6,10 etc. sowie Copolymere, von MF- und UF-Harzen, von heißvernetzbaren und thermoplastischen Acrylharzen und von Polyurethanen eingesetzt.

Entsprechende stabilisierte Massen sind somit eine Vorzugsform der vorher erwähnten und beanspruchten Massen. Bevorzugt werden Lacke aus derartigen Polymeren stabilisiert.

Die Stabilisatoren der Formel I können nach den herkömmlichen Methoden in das organische Material eingearbeitet werden, so z. B. in jeder beliebigen Phase während der Herstellung von geformten Produkten. Sie können zum Beispiel als Flüssigkeit, als Pulver, Suspensionen oder Emulsionen oder Lösungen zum Polymer gemischt werden, welches als Pulver, Schmelze, Lösung, Suspension oder Emulsion vorliegen kann.

Die stabilisierten Mischungen der Erfindung können gegebenenfalls 0,1 bis 5 Gew.-%, vorzugsweise 0,5 bis 3 Gew.-%, bezogen auf das Polymer, der üblichen Zusatzstoffe enthalten, insbesondere Antioxydantien, Lichtstabilisatoren, oder deren Gemische. Beispiele für solche Zusatzstoffe sind:

1. Antioxidantien

1.1. Alkylierte Monophenole
2,6-Di-tert.butyl-4-methylphenol
2-Tert.butyl-4,6-dimethylphenol
2,6-Di-tert.butyl-4-äthylphenol
2,6-Di-tert.butyl-4-n-butylphenol

2,6-Di-tert.butyl-4-i-butylphenol
2,6-Di-cyclopentyl-4-methylphenol
2-(α-Methylcyclohexyl)-4,6-dimethylphenol
2,6-Di-octadecyl-4-methylphenol
2,4,6-Tri-cyclohexylphenol
2,6-Di-tert.butyl-4-methoxymethylphenol

1.2. Alkylierte Hydrochinone
2,6-Di-tert.butyl-4-methoxyphenol
2,5-Di-tert.butyl-hydrochinon
2,5-Di-tert.amyl-hydrochinon
2,6-Diphenyl-4-octadecyloxyphenol

1.3. Hydroxylierte Thiodiphenyläther
2,2'-Thio-bis-(6-tert.butyl-4-methylphenol)
2,2'-Thio-bis-(4-octylphenol)
4,4'-Thio-bis-(6-tert.butyl-3-methylphenol)
4,4'-Thio-bis-(6-tert.butyl-2-methylphenol)

1.4. Alkyliden-Bisphenole
2,2'-Methylen-bis-(6-tert.butyl-4-methylphenol)
2,2'-Methylen-bis-(6-tert.butyl-4-äthylphenol)
2,2'-Methylen-bis-[4-methyl-6-(α-methylcyclohexyl)-phenol]
2,2'-Methylen-bis-(4-methyl-6-cyclohexylphenol)
2,2'-Methylen-bis-(6-nonyl-4-methylphenol)
2,2'-Methylen-bis-(4,6-di-tert.butylphenol)
2,2'-Äthyliden-bis-(4,6-di-tert.butylphenol)
2,2'-Äthyliden-bis-(6-tert.butyl-4-isobutylphenol)
4,4'-Methylen-bis-(2,6-di-tert.butylphenol)
4,4'-Methylen-bis-(6-tert.butyl-2-methylphenol)
1,1-Bis-(5-tert.butyl-4-hydroxy-2-methylphenyl)-butan
2,6-Di-(3-tert.butyl-5-methyl-2-hydroxybenzyl)-4-methylphenol
1,1,3-Tris-(5-tert.butyl-4-hydroxy-2-methylphenyl)-butan
1,1-Bis-(5-tert.butyl-4-hydroxy-2-methylphenyl)-3-n-dodecylmecaptobutan
Äthylenglycol-bis-[3,3-bis-(3'-tert.butyl-4'-hydroxyphenyl)-butyrat]
Di-(3-tert.butyl-4-hydroxy-5-methylphenyl)-dicyclopentadien
Di-[2-(3'-tert.butyl-2'-hydroxy-5'-methyl-benzyl)-6-tert.butyl-4-methyl-phenyl]-terephthalat.

1.5. Benzylverbindungen
1,3,5-Tri-(3,5-di-tert.butyl-4-hydroxybenzyl)-2,4,6-trimethylbenzol
Di-(3,5-di-tert.butyl-4-hydroxybenzyl)-sulfid
3,5-di-tert.butyl-4-hydroxybenzyl-mercaptoessigsäure-isooctylester
Bis-(4-tert.butyl-3-hydroxy-2,6-dimethylbenzyl)dithiol-terephthalat
1,3,5-Tris-(3,5-di-tert.butyl-4-hydroxybenzyl)-isocyanurat
1,3,5-Tris-(4-tert.butyl-3-hydroxy-2,6-dimethylbenzyl)-isocyanurat
3,5-Di-tert.butyl-4-hydroxybenzyl-phosphonsäure-dioctadecylester
3,5-Di-tert.butyl-4-hydroxybenzyl-phosphonsäure-monoäthylester, Calcium-salz.

1.6. Acylaminophenole
4-Hydroxy-laurinsäureanilid
4-Hydroxy-stearinsäureanilid
2,4-Bis-octylmercapto-6-(3,5-di-tert.butyl-4-hydroxyanilino)-S-triazin

1.7. Ester der β-(3,5-Di-tert.butyl-4-hydroxyphenyl)-propionsäure mit ein- oder mehrwertigen Alkoholen, wie z. B. mit

| | |
|---|---|
| Methanol | Diäthylenglycol |
| Octadecanol | Triäthylenglycol |
| 1,6-Hexandiol | Pentaerythrit |
| Neopentylglycol | Tris-hydroxyäthyl-isocyanurat |
| Thiodiäthylenglycol | Di-hydroxyäthyl-oxalsäurediamid |

1.8. Ester der β(5-tert.butyl-4-hydroxy-3-methylphenyl)-propionsäure mit ein- oder mehrwertigen Alkoholen, wie z. B. mit

| | |
|---|---|
| Methanol | Diäthylenglycol |
| Octadecanol | Triäthylenglycol |
| 1,6-Hexandiol | Pentaerythrit |
| Neopentylglycol | Tris-hydroxyäthyl-isocyanurat |
| Thiodiäthylenglycol | Di-hydroxyäthyl-oxalsäurediamid |

1.9. Amide der $\beta$-(3,5-Di-tert.butyl-4-hydroxyphenyl)-propionsäure, wie z. B.

N,N'-Di-(3,5-di-tert.butyl-4-hydroxyphenylpropionyl)-hexamethylendiamin
N,N'-Di-(3,5-di-tert.butyl-4-hydroxyphenylpropionyl)-trimethylendiamin
N,N'-Di-(3,5-di-tert.butyl-4-hydroxyphenylpropionyl)-hydrazin

2. UV-Absorber und Lichtschutzmittel

2.1. 2-(2'-Hydroxyphenyl)-benztriazole, wie z. B. das 5'-Methyl-, 3',5'-Di-tert.butyl-, 5'-Tert.butyl-, 5'-(1,1,3,3-Tetramethylbutyl)-, 5-Chlor-3',5'-di-tert.butyl-, 5-Chlor-3'-tert.butyl-5'-methyl-, 3'-sec.Butyl-5'-tert.butyl-, 4'Octoxy-, 3',5'-Di-tert.amyl-Derivat.

2.2. 2-Hydroxybenzophenone, wie z. B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z. B. 4-Tert.butyl-phenylsalicylat, Phenylsalicylat, Octylphenylsalicyalat, Dibenzoylresorcin, Bis-(4-tert.butylbenzoyl)-resorcin, Benzoylresorcin, 3,5-Di-tert.butyl-4-hydroxybenzoesäure-2,4-di-tert.butyl-phenylester.

2.4. Acrylate, wie z. B. $\alpha$-Cyan-$\beta$,$\beta$-diphenylacrylsäure-äthylester bzw. -isooctylester, $\alpha$-Carbomethoxy-zimtsäuremethylester, $\alpha$-Cyano-$\beta$-methyl-p-methoxy-zimtsäuremethylester bzw. -butylester, $\alpha$-Carbomethoxy-p-methoxy-zimtsäure-methylester. N-($\beta$-Carbomethoxy-$\beta$-cyanovinyl)-2-methyl-indolin.

2.5. Nickelverbindungen, wie z. B. Nickelkomplexe des 2,2'-Thio-bis-[4-(1,1,3,3-tetramethylbutyl)-phenols], wie der 1 : 1- oder der 1 : 2-Komplex, gegebenenfalls mit zusätzlichen Liganden wie n-Butylamin, Triäthanolamin oder N-Cyclohexyl-diäthanolamin, Nickeldibutyldithiocarbamat, Nickelsalze von 4-Hydroxy-3,5-di-tert.butylbenzyl-phosphonsäure-monoalkylestern wie vom Methyl- oder Äthylester, Nickelkomplexe von Ketoximen wie von 2-Hydroxy-4-methyl-phenyl-undecyl-ketonoxim, Nickelkomplexe des 1-Phenyl-4-lauroyl-5-hydroxy-pyrazols, gegebenenfalls mit zusätzlichen Liganden.

2.6. Sterisch gehinderte Amine, wie z. B.
Bis-(2,2,6,6-tetramethyl-piperidyl)-sebacat
Bis-(1,2,2,6,6-pentamethylpiperidyl)-sebacat
n-Butyl-3,5-di-tert.butyl-4-hydroxybenzyl-malonsäure-bis(1,2,2,6,6-pentamethylpiperidyl)-ester
Kondensationsprodukt aus 1-Hydroxyäthyl-2,2,6,6-Tetramethyl-4-hydroxypiperidin und Bernsteinsäure
Kondensationsprodukt aus N,N'-(2,2,6,6-Tetramethylpiperidyl)-hexamethylendiamin und 4-tert.octylamino-2,6-dichlor-1,3,5-s-triazin. Tris-(2,2,6,6-tetramethylpiperidyl)-nitrilotriacetat.

2.7. Oxalsäurediamide, wie z. B. 4,4'-Di-octyloxy-oxanilid, 2,2'-Di-octyloxy-5,5'-di-tert.butyl-oxanilid, 2,2'-Di-dodecyloxy-5,5'-di-tert.butyl-oxanilid, 2-Äthoxy-2'-äthyl-oxanilid, N,N'-Bis-(3-di-methylaminopropyl)-oxalamid, 2-Äthoxy-5-tert.butyl-2'-äthyl-oxanilid und dessen Gemisch mit 2-Äthoxy-2'-äthyl-5,4'-di-tert.butyl-oxanilid, Gemische von ortho- und para-Methoxy-sowie von o- und p-Äthoxy-di-substituierte Oxaniliden.

3. Metalldesaktivatoren, wie z. B. N,N'-Diphenyloxalsäurediamid, N-Salicylal-N'-salicyloylhydrazin, N,N'-Bis-salicyloylhydrazin, N,N'-Bis-(3,5-di-tert.butyl-4-hydroxyphenylpropionyl)-hydrazin, 3-Salicyloylamino-1,2,4-triazol, Bis-benzyliden-oxalsäuredihydrazid.

4. Phosphite und Phosphonite, wie z. B.
Triphenylphosphit, Diphenylalkylphosphite, Phenyldialkylphosphite, Tri-(nonylphenyl)-phosphit, Trilaurylphosphit, Trioctadecylphosphit, Distearyl-pentaerythrit-diphosphhit, Tris-(2,4-di-tert.butylphenyl)-phosphit, Diisodecyl-pentaerythrit-diphosphit, Di-(2,4-di-tert.butylphenyl)-pentaerythrit-diphosphit, Tristearyl-sorbit-triphosphit, Tetrakis-(2,4-di-tert.butylphenyl)-4,4'-biphenylen-diphosphonit.

5. Peroxidzerstörende Verbindungen, wie z. B.
Ester der $\beta$-Thio-di-propionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-($\beta$-dodecylmercapto)-propionat.

6. Polyamidstabilisatoren, wie z. B.
Kupfersalze in Kombination mit Jodiden und/oder Phosphorverbindungen und Salze des zweiwertigen Mangans.

7. Basische Co-Stabilisatoren, wie z. B.
Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrennzcatechinat.

8. Nukleierungsmittel, wie z. B.
4-tert.Butylbenzoesäure, Adipinsäure, Diphenylessigsäure.

9. Füllstoffe und Verstärkungsmittel, wie z. B.
Calciumcarbonate, Silikate, Glasfasern, Asbest, Talk, Kaolin, Glimmer, Bariumsulfat, Metalloxide und -hydroxide, Ruß, Graphit.

10. Sonstige Zusätze, wie z. B.
Weichmacher, Gleitmittel, Emulgatoren, Pigmente, Optische Aufheller, Flammschutzmittel, Antistatika, Treibmittel.

Von besonderem Interesse ist die Stabilisierung der duroplastischen und thermoplastischen Acrylharze, welche für Autolackierungen verwendet werden. Diese Stoffe sind in »Encyclopedia of Polymer Science and Technology«, Interscience Publisher, New York, Band 1 (1964), auf Seiten 273 bis 276 und Band 13 (1970), auf Seiten 530 bis 532 beschrieben, ferner in »Understanding Paint« von W. R. Fuller, in American Paint Journal, St. Louis, 1965, Seiten 124 bis 135.

Acrylharzlacke, welche gemäß der Erfindung gegen die Einwirkung von Licht, Sauerstoff und Feuchtigkeit stabilisiert werden, sind die üblichen Einbrennlacke, wie z. B. in H. Kittel's »Lehrbuch der Lacke und Beschichtungen«, Band 1, Teil 2, Seite 735 und 742 (Berlin, 1972), und in H. Wagner, H. F. Sarx, »Lackkunstharze«, Seite 229 bis 235 beschrieben.

Von besonderem Interesse ist die Stabilisierung von Metalleffektlacken auf der Basis von heißvernetzbarem Acrylharz mit den erfindungsgemäßen Verbindungen. Aus diesen Harzen hergestellter, unstabilisierter Metalleffektlack ist trotz der ausgezeichneten physikalischen und chemischen Eigenschaften ungeeignet, da das UV-Licht die Deckschicht ungehindert passieren kann und in der unteren Schicht zur Rißbildung führt. Andere Lacke und heißvernetzbare Beschichtungen auf der Basis von Acrylharz, Melaminharz, Alkydharz, Polyesterharz, Epoxyharz oder deren Gemischen können ebenfalls mit den erfindungsgemäßen Verbindungen wirksam stabilisiert werden.

Um die Metalleffektwirkung zu erzielen, verwendet man die üblichen Aluminium-Pigmente in 1 bis 10 Gew.-%, bezogen auf das lösungsmittelfreie Bindemittel (Lackharz). Das Auftragen der stabilisierten Lacke kann nach den herkömmlichen Einschicht- oder Zweischicht-Verfahren geschehen. Im letzteren Fall wird der das Pigment enthaltende Vorlegelack zuerst aufgetragen und nachher mit Klarlack überzogen.

Weitere Zusatzstoffe, die im Lack enthalten sein können, sind andere übliche Lichtschutzmittel, phenolische Antioxydantien, Pigmente, Farbstoffe, Metalldesaktivatoren etc.

Einen besonders wirkungsvoll stabilisierten Lack stellt ein solcher folgender Zusammensetzung dar:

a) heißvernetzbares Acrylharz und
b) 0,1 bis 5 Gew.-%, bezogen auf das Harz einer Verbindung der Formel I oder eines Gemisches von Verbindungen der Formel I, und
c) 0,1 bis 5 Gew.-%, bezogen auf das Harz eines sterisch gehinderten Amin-Stabilisators.

Bevorzugt enthält der Lack 0,5 bis 2 Gew.-%, bezogen auf das Harz, von der Komponente b) und 0,5 bis 2 Gew.-%, bezogen auf das Harz, von der Komponente c).

Geeignete sterisch gehinderte Amine sind z. B. solche, wie sie in der DE-OS 2 500 134, in der US 4 110 304 oder im »Taschenbuch der Kunststoff-Additive« von R. Gächter und H. Müller (Hanser Verlag, München, 1979) beschrieben sind. Insbesondere geeignet sind

Bis-(1,2,2,6,6-pentamethyl-4-piperidyl)-sebacat oder

Bis-(1,2,2,6,6-pentamethyl-4-piperidyl)-2-n-butyl-2-(3,5-di-tert.-butyl-4-hydroxy-benzyl)-malonat.

Die Kombination von sterisch gehinderten Aminen und den erfindungsgemäßen Stabilisatoren ermöglicht sowohl eine ausgezeichnete Glanzerhaltung bei Bewitterung als auch Beständigkeit gegen das Abblättern metallisierter Einbrennlacke für Automobil-Decklackierungen.

Die erfindungsgemäßen Verbindungen der Formel I unterscheiden sich in vier hervorragenden Eigenschaften von den strukturell nahestehenden Benztriazolverbindungen des Standes der Technik. Sie sind:

1. schwerflüchtig
2. gut löslich in den Lösungsmitteln, welche für Kunststoffüberzüge verwendet werden,
3. besser verträglich mit Polyolefinen oder Vinylpolymeren
4. sie zeigen, vor allem als Gemische eingesetzt, geringe Kristallisationsneigung.

Die Schwerflüchtigkeit, insbesondere wenn mit guter Substratverträglichkeit und Löslichkeit kombiniert, erlaubt die unproblematische Einarbeitung der erfindungsgemäßen Verbindungen in das Polymer, wo sie auch nach der Hochtemperatur-Verarbeitung verbleiben und daher dem Endprodukt die gewünschte Stabilität verleihen.

Die größere Substratverträglichkeit der erfindungsgemäßen Verbindungen zusammen mit der bereits diskutierten Schwerflüchtigkeit gestattet die Verarbeitung und Verwendung der stabilisierten Produkte bei höheren Temperaturen, sowie eine verlängerte Gebrauchsdauer der Produkte bei üblichen Temperaturen. Dadurch wird das unerwünschte Ausschwitzen des Stabilisators während der Verarbeitung verhindert, was sonst häufig zu kostspieligen Schädigungen der Produktionsanlage führt.

Weiterer Gegenstand der Erfindung ist auch noch stabilisiertes, organisches Material, welches dadurch gekennzeichnet ist, daß es als Photomaterial vorliegt oder Teil eines Photomaterials ist, wobei das Photomaterial, vorzugsweise in Deckschichten, 0,1 bis 5 Gew.-%, bezogen auf das Photomaterial ohne Stabilisator, einer erfindungsgemäßen Verbindung enthält.

Die folgenden Beispiele erläutern die Erfindung.

## Beispiel 1

37,1 g Azofarbstoff (Smp. 201°C) erhalten durch Kupplung von diazotiertem o-Nitranilin mit 2-(3-tert.-Butyl-4-hydroxyphenyl)-propionsäure werden in 200 ml 2-n Natronlauge eingetragen. Dazu gibt man 30 g Zinkstaub und läßt dann innerhalb einer Stunde 50 ml 10-n Natronlauge zutropfen, wobei die Temperatur unterhalb 45°C gehalten wird. Anschließend erwärmt man 1 Stunde auf 85 bis 90°C, filtriert bei dieser Temperatur vom Zinkschlamm ab und säuert das Filtrat mit konzentrierter Salzsäure an. Der dabei ausfallende Niederschlag wird abgenutscht, mit Wasser gewaschen, getrocknet und aus Ligroin umkristallisiert. Man erhält das 2-[2-Hydroxy-3-tert.-butyl-5-(2-carboxyäthyl)-phenyl]-benztriazol (Verbindung 1) vom Smp. 195°C.

Verwendet man anstelle des obengenannten Azofarbstoffes den Azofarbstoff (Smp. 192°C) erhalten durch Kupplung von diazotiertem p-chloro-o-nitranilin mit 2-(3-tert.-Butyl-4-hydroxyphenyl)-propionsäure und verfährt man wie oben beschrieben, so erhält man das 2-[2-Hydroxy-3-tert.-butyl-5-(2-carboxyäthyl)-phenyl]-5-chlorbenztriazol (Verbindung 2) mit Smp. 192°C.

## Beispiel 2

33,9 g 2-[2-Hydroxy-3-tert.-butyl-5-(2-carboxyäthyl)-phenyl]-benztriazol (Verbindung 1) werden in 200 ml Toluol suspendiert. Nach der Zugabe von 2 ml Dimethylformamid und 20 g Thionylchlorid wird das Reaktionsgemisch langsam auf 80°C erwärmt und bei dieser Temperatur gerührt bis die Schwefeldioxid- und Salzsäureentwicklung aufhört. Überschüssiges Thionylchlorid und Toluol werden dann im Vakuum abdestilliert und der erhaltene Rückstand aus Hexan umkristallisiert. Man erhält das 2-[2-Hydroxy-3-tert.-butyl-5-(2-chlorocarbonyläthyl)-phenyl]-benztriazol (Verbindung 3) mit Smp. 104 bis 106°C.

Verfährt man genau gleich mit 2-[2-Hydroxy-3-tert.-butyl-5-(2-carboxyäthyl)-phenyl]-5-chlorbenztriazol, so erhält man das 2-[2-Hydroxy-3-tert.-butyl-5-(2-chlorocarbonyläthyl)-phenyl]-5-chlorbenztriazol (Verbindung 4) mit Smp. 102 bis 105°C.

Beispiel 3

33,9 g 2-[2-Hydroxy-3-tert.-butyl-5-(2-carboxyäthyl)-phenyl]-benztriazol (Verbindung 1) werden in 150 ml Methanol und 1 ml konzentrierter Schwefelsäure 3 Stunden am Rückfluß gekocht. Beim Abkühlen der Reaktionslösung kristallisiert das 2-[2-Hydroxy-3-tert.-butyl-5-(2-carbomethoxyätyl)-phenyl]-benztriazol (Verbindung 5) vom Smp. 121 bis 22° C aus.

Verfährt man mit 2-[2-Hydroxy-3-tert.-butyl-5-(2-carboxyäthyl)-phenyl]-5-chlorbenztriazol (Verbindung 2) genau gleich, so erhält man das 2-[2-Hydroxy-3-tert.butyl-5-(2-carbomethoxyäthyl)-phenyl]-5-chlorbenztriazol (Verbindung 6) mit Smp. 112 bis 113° C.

Verwendet man anstelle von Methanol die gleiche Menge Äthanol, n-Butanol, iso-Butanol, Allylalkohol oder Chlorpropanol und läßt ca. 3—6 Stunden bei einer Temperatur von 70—75° C mit 1 ml konz. Schwefelsäure reagieren, dampft das Reaktionsgemisch am Rotovapor ein und kristallisiert den Rückstand aus Hexan bzw. Ligroin aus, dann erhält man

2-[2-Hydroxy-3-tert.butyl-5-(2-carboäthoxyäthyl)-phenyl]-benzotriazol.
Smp.: 96—98° C (Verb. 7)
2-[2-Hydroxy-3-tert.butyl-5-(2-carboäthoxyäthyl)-phenyl]-5-chlorbenzotriazol.
Smp.: 100—101° C (Verb. 8)
2-[2-Hydroxy-3-tert.butyl-5-(2-carbo-n-butoxyäthyl)-phenyl)]-benzotriazol.
Smp.: 55—57° C (Verb. 9)
2-[2-Hydroxy-3-tert.butyl-5-(2-carbo-n.butoxyäthyl)-phenyl]-5-chlorbenzotriazol.
Smp.: 63—64° C (Verb. 10)
2-[2-Hydroxy-3-tert.butyl-5-(2-carbo-isobutoxyäthyl)-phenyl]-benzotriazol.
Smp.: 86—87° C (Verb. 11)
2-[2-Hydroxy-3-tert.butyl-5-(2-carbo-iso.butoxyäthyl)-phenyl]-5-chlorbenzotriazol.
Smp.: 90—91° C (Verb. 12)
2-[2-Hydroxy-3-tert.butyl-5-(2-carboallyloxyäthyl)-phenyl]-benzotriazol.
Smp.: 120—121° C (Verb. 13)
2-[2-Hydroxy-3-tert.butyl-5-(2-carbo-γ-chlorpropyloxyäthyl)-phenyl]-benzotriazol.
Smp.: 82—84° C (Verb. 14).

Beispiel 4

35,3 g 2-[2-Hydroxy-3-tert.-butyl-5-(2-carbomethoxyäthyl)-phenyl]-benztriazol (Verbindung 3) werden in 200 ml Xylol mit 12 g Cyclohexanol und 1 g Tetrabutylorthotitanat ca. 12 Stunden bei 130°C gerührt bis kein Methanol mehr abdestilliert. Nach dem Abkühlen auf Raumtemperatur wird die Reaktionslösung mit 200 ml einer gesättigten, wäßrigen Natriumbicarbonatlösung ausgeschüttelt, mit Wasser gewaschen, über Natriumsulfat getrocknet und am Vakuum eingedampft. Durch Kristallisation des Eindampfrückstandes aus Hexan erhält man das 2-[2-Hydroxy-3-tert.-butyl-5-(2-carbocyclohexyloxyäthyl)-phenyl]-benztriazol (Verbindung 15) mit Smp. 86 bis 87° C.

Verwendet man anstelle von Cyclohexanol eine entsprechende Menge n-Octanol, 2-Äthyl-hexanol, iso-Decanol oder n-Dodecanol und verfährt man im übrigen wie oben beschrieben, so erhält man das 2-[2-Hydroxy-3-tert.-butyl-5-(2-carbooctyloxyäthyl)-phenyl]-benztriazol (Verbindung 16) als gelbliches Harz, das mit der Zeit zu eine wachsartigen Masse erstarrt, bzw. das 2-{2-Hydroxy-3-tert-butyl-5-[2-carbo-(2-äthylhexyloxy)-äthyl]-phenyl}-benztriazol (Verbindung 17) als leicht gelbes, dickflüssiges Harz, bzw. das 2-[2-Hydroxy-3-tert.-butyl-5-(2-carbo-iso-decyloxyäthyl)-phenyl]-benztriazol (Verbindung 18) als leicht gelbliches Harz, bzw. das 2-[2-Hydroxy-3-tert.-butyl-5-(2-carbododecyloxyäthyl)-phenyl]-benztriazol (Verbindung 19) mit Smp. 51 bis 52° C.

Beispiel 5

37,4 g 2-[2-Hydroxy-3-tert.-butyl-5-(2-carboxyäthyl)-phenyl]-5-chlorbenztriazol (Verbindung 2) werden mit 20 g 1-Dodecanol und 2 ml Phosphorsäure in 400 ml Toluol 12 Stunden am Wasserabscheider gekocht. Nach dem Abkühlen wird die Reaktionslösung mit Wasser neutralgewaschen und dann am Vakuum eingedampft. Nach Umkristallisation des Eindampfrückstandes aus Isopropanol erhält man das 2-[2-Hydroxy-3-tert.-butyl-5-(2-carbododecyloxyäthyl)-phenyl]-5-chlorbenztriazol (Verbindung 20) mit Smp. 55 bis 56° C.

Verwendet man anstelle von 1-Dodecanol eine entsprechende Menge a) 1-Octanol, b) 2-Äthylhexanol, c) Glycerin, d) Cyclohexanol, e) Triäthylenglykol, f) tert.Amylalkohol, g) Hexandiol oder h) Cyclohexylcarbinol und verfährt im übrigen wie oben beschrieben, so erhält man folgende Produkte:

a) 2-[2-Hydroxy-3-tert.butyl-5-(2-carbooctyloxyäthyl)-phenyl]-5-chlorbenztriazol (Verbindung 21),

b) 2-{2-Hydroxy-3-tert.butyl-5-[2-carbo-(2-äthylhexyloxy)-äthyl]-phenyl}-5-chlorbenztriazol (Verbindung 22),

c) eine Verbindung der Formel

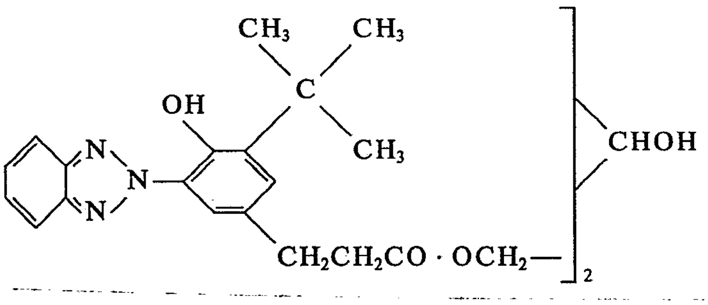

(glasiges Harz; Ep. 94—97° C) (Verb. 23)

d) 2-[2-Hydroxy-3-tert.butyl-5-(2-carbocyclohexyloxyäthyl)-phenyl]-5-chlorbenzotriazol (Verbindung 24); Smp. 84—86° C (Hexan),

e) eine Verbindung der Formel

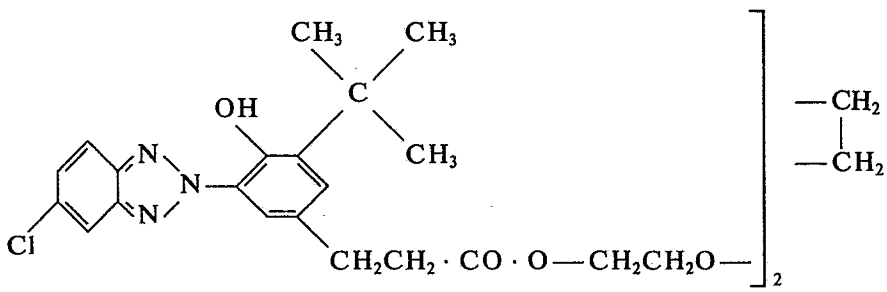

Smp. 103—105° C (Toluol) (Verb. 25)

f) 2-[2-Hydroxy-3-tert.butyl-5-(2-carbo-tert.amyloxyäthyl)-phenyl]-benzotriazol; Smp. 101—103° C (Hexan) (Verb. 26),
2-[2-Hydroxy-3-tert.butyl-5-(2-carbo-tert.amyloxyäthyl)-phenyl]-chlorbenzotriazol; Smp. 94—95° C (Verb. 27),

g) 2-[2-Hydroxy-3-tert.butyl-5-(2-carbo-(6-hydroxyhexyloxy)-äthyl)-phenyl]-benztriazol; Smp. 57—59° C (Verb. 29).
2-[2-Hydroxy-3-tert.butyl-5-(2-carbo-(6-hydroxyhexyloxy)-äthyl)-phenyl]-chlorbenzotriazol, ein Harz (Verb. 30).

EA.:

| | | | | |
|---|---|---|---|---|
| Ber.: | C=63,50% | H=6,80% | N=8,87% | Cl=7,48 |
| Gef.: | C=63,56% | H=6,97% | N=8,95% | Cl=7,33% |

h) 2-[2-Hydroxy-3-tert.butyl-5-(2-carbocyclohexylmethyloxyäthyl)-phenyl]-5-chlorbenztriazol; Smp. 100—101° C (Verb. 28).

mit Hexandiol-Verdopplungen:

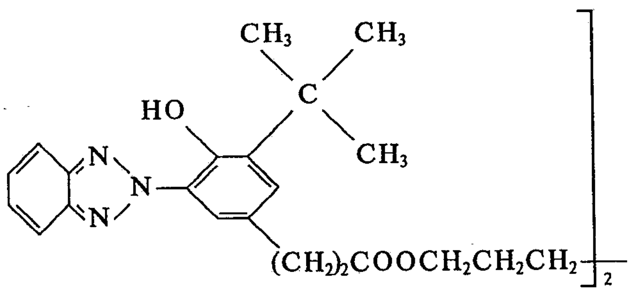

Smp.: 106—108° C (Essigester) (Verb. 31)

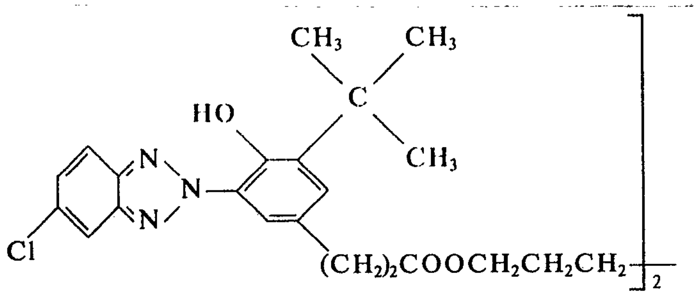

Smp.: 91—93° C (Hexan) (Verb. 32)

Beispiel 6

33,9 g 2-[2-Hydroxy-3-tert.-butyl-5-(2-carboxyäthyl)-phenyl]-benztriazol (Verbindung 1) werden mit 9,0 g Cyclohexenoxid in 300 ml Xylol 12 Stunden unter Stickstoff am Rückfluß gekocht. Das Lösungsmittel wird im Vakuum eingedampft und der Rückstand aus Ligroin kristallisiert. Man erhält das 2-{2-Hydroxy-3-tert.-butyl-5-[2-carbo-(2-hydroxycyclohexyloxy)-äthyl]-phenyl}-benztriazol (Verbindung 33) mit Smp. 118 bis 119° C.

Verwendet man anstelle von Cyclohexenoxid die folgenden Epoxide und Glycidylverbindungen und verfährt im übrigen wie oben beschrieben, so erhält man die nachstehenden Benzotriazole:

Epichlorhydrin:

2-[2-Hydroxy-3-tert.butyl-5-(2-carbo-(2-hydroxy-3-chlorpropyloxy)-äthyl)-phenyl]-benzotriazol. Smp.: 69—71° C (Cyclohexan) (Verb. 34)

2-[2-Hydroxy-3-tert.butyl-5-(2-carbo-(2-hydroxy-3-chlorpropyloxy)-äthyl)-phenyl]-5-chlorbenzotriazol. Smp.: 90—92° C (Cyclohexan) (Verb. 35)

Butylglycidyläther:

2-[2-Hydroxy-3-tert.butyl-5-(2-carbo-(2-hydroxy-4-oxaoctyloxy)-äthyl)-phenyl]-benzotriazol.Smp.: 42—43° C (Ligroin) (Verb. 36)

2-[2-Hydroxy-3-tert.butyl-5-(2-carbo-(2-hydroxy-4-oxaoctyloxy)-äthyl)-phenyl]-5-chlorbenzotriazol. Harz (Verb. 37)

EA.:

| | | | | |
|---|---|---|---|---|
| Ber.: | C=61,71% | H=7,17% | N=8,30% | Cl=7,01% |
| Gef.: | C=61,91% | H=6,83% | N=8,31% | Cl=7,27% |

Allylglycidyläther:

2-[2-Hydroxy-3-tert.butyl-5-(2-carbo-(2-hydroxy-3-allyloxypropoxy)-äthyl)-phenyl]-benzotriazol. Smp.: 52—53° C (Hexan) (Verb. 38)

2-[2-Hydroxy-3-tert.butyl-5-(2-carbo-(2-hydroxy-3-allyloxypropoxy)-äthyl)-phenyl]-5-chlorbenzotriazol. Harz (Verb. 39)

EA.:

| | | | | |
|---|---|---|---|---|
| Ber.: | C=61,53% | H=6,20% | N=8,61% | Cl=7,26% |
| Gef.: | C=61,60% | H=6,11% | N=8,71% | Cl=7,40% |

Cyclohexylglycidyläther:

2-[2-Hydroxy-3-tert.butyl-5-(2-carbo-(2-hydroxy-4-cyclohexyl-4-oxabutyloxy)-äthyl)-phenyl]-benzotriazol. Smp.: 69—70° C (Verb. 40)

2-[2-Hydroxy-3-tert.butyl-5-(2-carbo-(2-hydroxy-4-cyclohexyl-4-oxabutyloxy)-äthyl)-phenyl]-5-chlorbenzotriazol. Harz (Verb. 41)

EA.:

| | | | | |
|---|---|---|---|---|
| Ber.: | C=63,45% | H=6,85% | N=7,93% | Cl=6,69% |
| Gef.: | C=63,75% | H=6,81% | N=8,00% | Cl=7,01% |

Styroloxid:

2-[2-Hydroxy-3-tert.butyl-5-(2-carbo-(2-hydroxy-2-penyläthoxy)-äthyl)-phenyl]-benzotriazol. Smp.: 103—105° C (Acetonitril) (Verb. 42).

Phenylglycidyläther:
2-[2-Hydroxy-3-tert.butyl-5-(2-carbo-(2-hydroxy-3-phenoxypropyloxy)-äthyl)-phenyl]-benzotriazol. Smp.: 99—100° C (Verb. 43).

2-tert.Butylphenylglycidyläther:
2-[2-Hydroxy-3-tert.butyl-5-(2-carbo-(2-hydroxy-3-(2-tert.butylphenyl)oxy-propyloxy)-äthyl)-phenyl]-benzotriazol. Smp.: 54—55° C (Acetoniril) (Verb. 44).

4-Phenoxyphenylglycidyläther:
2-[2-Hydroxy-3-tert.butyl-5-(2-carbo-(2-hydroxy-3-(4-phenoxyphenyl)oxy-propyloxy)-äthyl)-phenyl]-benzotriazol. Smp.: 73—74° C (Hexan) (Verb. 45).

4-(1-Methyl-1-phenyläthyl)-phenylglycidyläther:
2-[2-Hydroxy-3-tert.butyl-5-(2-carbo-(2-hydroxy-3-(4-(1-methyl-1-phenyläthyl)phenyl)-propyloxy)-äthyl-)phenyl]-benzotriazol. Glasiges Harz Ep.: 43—48° C (Verb. 46).

Octylglycidyläther:
2-[2-Hydroxy-3-tert.butyl-5-(2-carbo-(2-hydroxy-3-octyloxypropyloxy)-äthyl)-phenyl]-5-chlorbenzotriazol. Harz (Verb. 47)

EA.:

| | | | | |
|---|---|---|---|---|
| Ber.: | C=64,32% | H=7,56% | N=7,50% | Cl=6,33% |
| Gef.: | C=64,42% | H=7,28% | N=7,32% | Cl=6,60% |

2-Äthylhexylglycidyläther:
2-[2-Hydroxy-3-tert.butyl-5-(2-carbo(2-hydroxy-3-(2-äthylhexyloxy)-propyloxy)-äthyl)-phenyl]-5-chlorbenzotriazol. Harz (Verb. 48)

EA.:

| | | | | |
|---|---|---|---|---|
| Ber.: | C=64,32% | H=7,56% | N=7,50% | Cl=6,33% |
| Gef.: | C=64,42% | H=7,49% | N=7,64% | Cl=6,83% |

Butandioldiglycidyläther

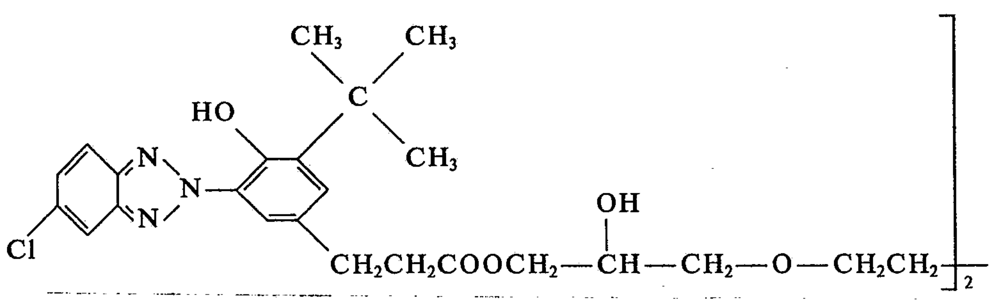

glasiges Harz, Ep.: 58—62° C (Verb. 49)

Bisphenol-A-diglycidyläther

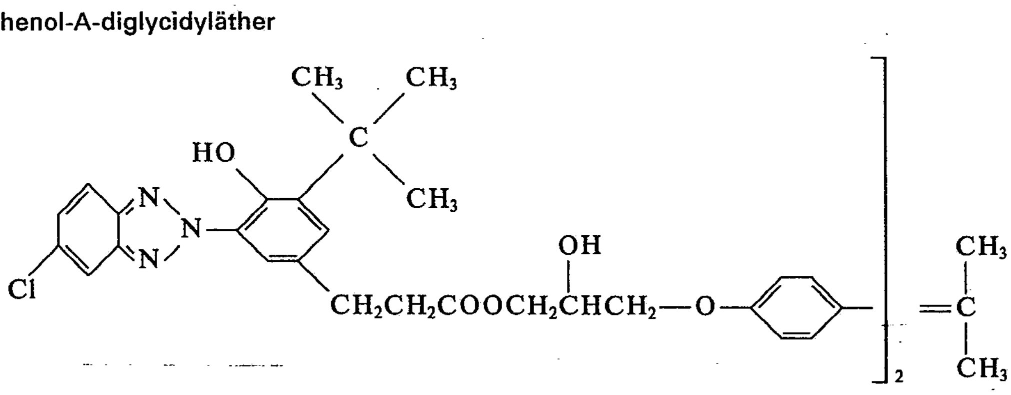

glasiges Harz, Ep.: 72—76° C (Verb. 50).

Beispiel 7

10,0 g Piperidin und 12,0 g Triäthylamin werden in 200 ml Toluol gelöst. Zu dieser Lösung tropft man bei 20 bis 25° C, in ca. 30 Minuten, unter gutem Rühren eine Lösung von 35,8 g 2-[2-Hydroxy-3-tert.-butyl-5-(2-chlorocarbonyläthyl)-phenyl]-benztriazol in 100 ml Toluol. Anschließend wird 1 Stunde bei 35 bis 40° C nachgerührt und die Reaktionslösung dann dreimal mit je 200 ml Wasser extrahiert. Die Toluollösung wird am Vakuum eingedampft und der Rückstand aus Ligroin kristallisiert. Man erhält das 2-[2-Hydroxy-3-tert.-butyl-5-(2-carbopiperidylamidoäthyl)-phenyl]-benztriazol (Verbindung 51) mit dem Smp. 102 bis 104° C.

Verwendet man anstelle von Piperidin eine äquivalente Menge Morpholin oder 2,6-Di-tert.-butyl-4-aminophenol und verfährt man im übrigen wie oben beschrieben, so erhält man das 2-[2-Hydroxy-3-tert.-butyl-5-(2-carbomorpholinamidoäthyl)-phenyl]-benztriazol (Verbindung 52) mit dem Smp. 177 bis 179° C, bzw. das 2-{2-Hydroxy-3-tert.-butyl-5-[2-carbo-(3,5-di-tert.-butyl-4-hydroxyanilido)-äthyl]-phenyl}-benztriazol (Verbindung 53) mit dem Smp. 217 bis 219° C.

Verwendet man Piperazin, so erhält man

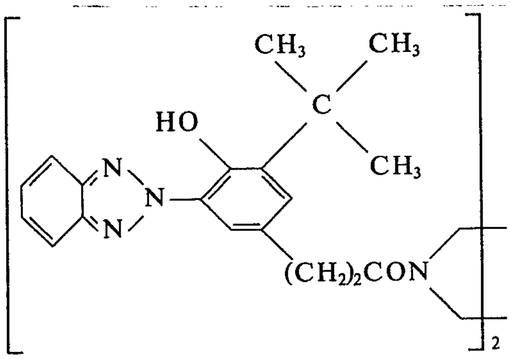

Smp. 265—266° C (Xylol) (Verb. 54)

verwendet man Diisooctylamin, so erhält man

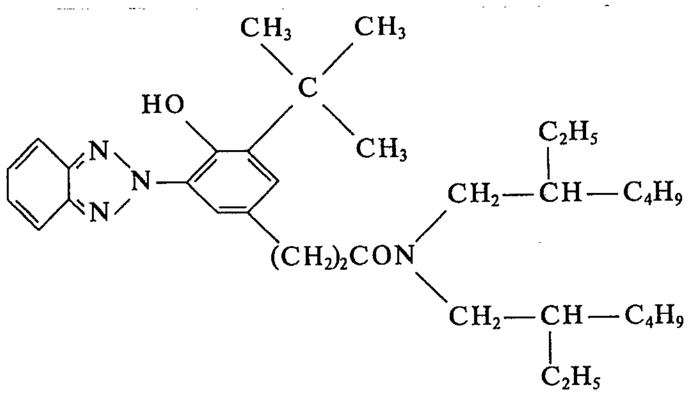

Harz (Verb. 55)

EA.:

| | | | |
|---|---|---|---|
| Ber.: | C = 74,69% | H = 9,67% | N = 9,96% |
| Gef.: | C = 74,74% | H = 9,64% | N = 10,11% |

Verwendet man Morpholin und setzt dieses mit 2-[2-Hydroxy-3-tert.butyl-5-(2-chlorocarbonyläthyl)-phenyl]-5-chlorbenzotriazol um, so erhält man

2-[2-Hydroxy-3-tert.butyl-5-(2-carbomorpholinamidoäthyl)-phenyl]-5-chlorbenzotriazol.

Smp.: 159—160° C (Cyclohexan) (Verb. 56).

Beispiel 8

33,9 g 2-[2-Hydroxy-3-tert.butyl-5-(2-carboxyäthyl)-phenyl]-benzotriazol werden in 100 ml Äthylenglykolmonomethyläther suspendiert, mit 1 ml konzentrierter Schwefelsäure versetzt und während ca. 20 Stunden unter Rühren auf 70—75° C erwärmt. Nach kurzer Zeit entsteht eine bräunlichgelbe klare Lösung. Nach beendeter Reaktionszeit wird die gesamte Lösung auf ca. 1,5 l destilliertes Wasser

gegossen und mit Essigsäureäthylester extrahiert. Die organische Phase wird noch zweimal mit destilliertem Wasser (bzw. mit 5%iger Kaliumcarbonatlösung, wenn noch wenig nicht reagierte Benzotriazolcarbonsäure vorhanden ist) gewaschen, mit Natriumsulfat sicc getrocknet und eingedampft. Der Rückstand wird aus Ligroin umkristallisiert. Man erhält 2-[2-Hydroxy-3-tert.butyl-5-(2-carbo-(2-methoxyäthoxy)-äthyl)-phenyl]-benzotriazol. Smp.: 86—87° C (Verb. 57).

Verwendet man zur Veresterung die entsprechende »Chlorbenzotriazolcarbonsäure«, so erhält man nach obigem Verfahren folgende Verbindung 2-[2-Hydroxy-3-tert.butyl-5-(2-carbo-(2-methoxyäthoxy)-äthyl)-phenyl]-5-chlorbenzotriazol.Smp.: 66—67° C (Verb. 58).

Verwendet man anstelle von Äthylenglykolmonomethyläther: a) Äthylenglykolmonoäthyläther oder b) Äthylenglykolmonobutyläther oder c) Diäthylenglykolmonoäthyläther oder d) Diäthylenglykolmonobutyläther und verfährt im übrigen genau gleich, wie oben beschrieben, so erhält man neue, teilweise flüssige viskose Benzotriazol-Öle und teilweise Benzotriazole, die erst nach Wochen oder Monaten kristallisieren.

a) 2-[2-Hydroxy-3-tert.butyl-5-(2-carbo-(2-äthoxyäthoxy)-äthyl)-phenyl]-benzotriazol. Smp.: 53—54° C (Verb. 59)
2-[2-Hydroxy-3-tert.butyl-5-(2-carbo-(2-äthoxyäthoxy)-äthyl)-phenyl]-5-chlorbenzotriazol Smp.: 45—47° C (Verb. 60)

b) 2-[2-Hydroxy-3-tert.butyl-5-(2-carbo-(2-butoxyäthoxy)-äthyl)-phenyl]-benzotriazol. Flüssiges Öl (Verb. 61)

Elementaranalyse (EA):

| | | | |
|---|---|---|---|
| Ber.: | C=68,31% | H=7,57% | N=9,56% |
| Gef.: | C=68,31% | H=7,60% | N=9,52% |

2-[2-Hydroxy-3-tert.butyl-5-(2-carbo-(2-butoxyäthoxy)-äthyl)-phenyl]-5-chlorbenzotriazol. Gelbliches Öl (Verb. 62)

EA.:

| | | | | |
|---|---|---|---|---|
| Ber.: | C=63,35% | H=6,81% | N=8,86% | Cl=7,48% |
| Gef.: | C=63,1% | H=6,7% | N=9,0% | Cl=7,6% |

c) 2-[2-Hydroxy-3-tert.butyl-5-(2-carbo-(3,6-dioxaoctyloxy)-äthyl)-phenyl]-benzotriazol. Smp.: 42—44° C (Verb. 63)
2-[2-Hydroxy-3-tert.butyl-5-(2-carbo-(3,6-dioxaoctyloxy)-äthyl)-phenyl]-5-chlorbenzotriazol. Gelbliches Öl (Verb. 64)

EA.:

| | | | | |
|---|---|---|---|---|
| Ber.: | C=61,28% | H=6,58% | N=8,58% | Cl=7,23% |
| Gef.: | C=61,27% | H=6,63% | N=8,69% | Cl=7,27% |

d) 2-[2-Hydroxy-3-tert.butyl-5-(2-carbo-(3,6-dioxadecyloxy)-äthyl)-phenyl]-benzotriazol. Smp.: 42—44° C (Verb. 65)
2-[2-Hydroxy-3-tert.butyl-5-(2-carbo-(3,6-dioxadecyloxy)-äthyl)-phenyl]-5-chlorbenzotriazol. Gelbliches, viskoses Öl (Verb. 66)

EA.:

| | | | | |
|---|---|---|---|---|
| Ber.: | C=62,60% | H=7,00% | N=8,11% | Cl=6,84% |
| Gef.: | C=62,70% | H=7,19% | N=8,00% | Cl=7,05% |

mit

e) Triäthylenglykol
2-[2-Hydroxy-3-tert.butyl-5-(2-carbo-(8-hydroxy-3,6-dioxaoctyloxy)-äthyl)-phenyl]-benzotriazol. Gelbes Harz (Verb. 67)

EA.:

| | | | |
|---|---|---|---|
| Ber.: | C=63,68% | H=7,05% | N=8,91% |
| Gef.: | C=63,80% | H=7,09% | N=9,12% |

2-[2-Hydroxy-3-tert.butyl-5-(2-carbo-(8-hydroxy-3,6-dioxaoctyloxy)-äthyl)-phenyl]-5-chlorbenzotriazol. Gelbes Öl (Verb. 68)

EA.:

| | | | | |
|---|---|---|---|---|
| Ber.: | C=59,34% | H=6,37% | N=8,30% | Cl=7,01% |
| Gef.: | C=59,42% | H=6,51% | N=8,50% | Cl=7,00% |

mit

f) Diäthylenglykol

2-[2-Hydroxy-3-tert.butyl-5-(2-carbo-(6-hydroxy-3-oxahexyloxy)-äthyl)-phenyl]-benzotriazol. Smp.: 74—76° C (Verb. 69)

2-[2-Hydroxy-3-tert.butyl-5-(2-carbo-(12-hydroxy-3,6,9-trioxadodecyloxy)-äthyl)-phenyl]-benzotriazol. Gelbes Öl (Verb. 70)

EA.:

Ber.: C=62,90% H=7,28% N=8,15%

Gef.: C=63,15% H=7,31% N=8,43%

mit

g) Tetraäthylenglykol

2-[2-Hydroxy-3-tert.butyl-5-(2-carbo-(12-hydroxy-3,6,9-trioxadodecyloxy)-äthyl)-phenyl]-5-chlorbenzotriazol. Viskoses Öl (Verb. 71)

EA.:

Ber.: C=58,96% H=6,60% N=7,64% Cl=6,44%

Gef.: C=58,98% H=6,86% N=7,88% Cl=6,47%

## Beispiel 9

35,3 g 2-[2-Hydroxy-3-tert.butyl-5-(2-carbomethyloxyäthyl)-phenyl]-benzotriazol (Verbindung 5) werden auf 130° C erhitzt und damit geschmolzen. Unter einem $N_2$-Strom läßt man langsam 20 ml 2-Äthyl-hexylamin (ca. 50% Überschuß der äquivalenten Menge) hinzutropfen. Nun destilliert langsam das sich bildende Methanol ab. Der Reaktionsverlauf wird durch Dünnschichtchromatographie verfolgt. Nach ca. 8 Stunden ist der Umsatz nahezu vollständig. Die Schmelze wird in Toluol warm gelöst und mit dem üblichen Adsorbtionsmittel wie Tonsil gereinigt. Man dampft die Toluollösung ein und kristallisiert den Rückstand um. Man erhält 2-[2-Hydroxy-3-tert.butyl-5-(2-carbo-(2-äthylhexyl)-amido-äthyl)-phenyl]-benzotriazol. Smp.: 112—113° C (Acetonitril) (Verb. 72)

Wählt man anstelle von 2-Äthyl-hexylamin die folgenden Amine und verfährt wie in Beispiel 9 beschrieben, so erhält man

mit Dodecylamin

2-[2-Hydroxy-3-tert.butyl-5-(2-carbododecylamidoäthyl)-phenyl]-benzotriazol. Smp.: 109—111° C (Hexan) (Verb. 73)

mit Cyclohexylamin

2-[2-Hydroxy-3-tert.butyl-5-(2-carbocyclohexylamidoäthyl)-phenyl]-benzotriazol. Smp.: 172—173° C (Toluol) (Verb. 74)

mit 3-(N-Morpholinyl)-propylamin

2-[2-Hydroxy-3-tert.butyl-5-(2-carbo-(3-(N-morpholin]1)propyl)-amido-äthyl-)phenyl]-benzotriazol.Smp.: 104—106° C (Acetonitril) (Verb. 75)

mit 3-(Octyloxy)propylamin

2-[2-Hydroxy-3-tert.butyl-5-(2-carbo-(3-(octyloxy)propylamido)äthyl)-phenyl]-benzotriazol. Smp.: 61—63° C (Hexan) (Verb. 76)

2-[2-Hydroxy-3-tert.butyl-5-(2-carbo-(3-(octyloxy)propylamido)äthyl)-phenyl]-5-chlorbenzotriazol. Smp.: 82—83° C (Hexan) (Verb. 77)

mit 3-Hydroxypropylamin

2-[2-Hydroxy-3-tert.butyl-5-(2-carbo-(3-hydroxypropyl)amidoäthyl)-phenyl]-benzotriazol. Smp.: 132—135° C (Acetonitril) (Verb. 78)

mit tert.Octylamin (≡ 1,1,3,3-Tetramethylbutylamin)

2-[2-Hydroxy-3-tert.butyl-5-(2-carbo-(1,1,3,3-tetramethyl)-butylamidoäthyl)-phenyl]-benzotriazol. Smp.: 157—158° C (Essigester) (Verb. 79)

mit 5-Hydroxy-3-oxapentylamin

2-[2-Hydroxy-3-tert.butyl-5-(2-carbo-(5-hydroxy-3-oxa)-pentylamidoäthyl)-phenyl]-benzotriazol. Smp.: 115—117° C (Acetonitril) (Verb. 80)

mit 3-[4-(3-Aminopropyloxy)-butyloxy]-propylamin

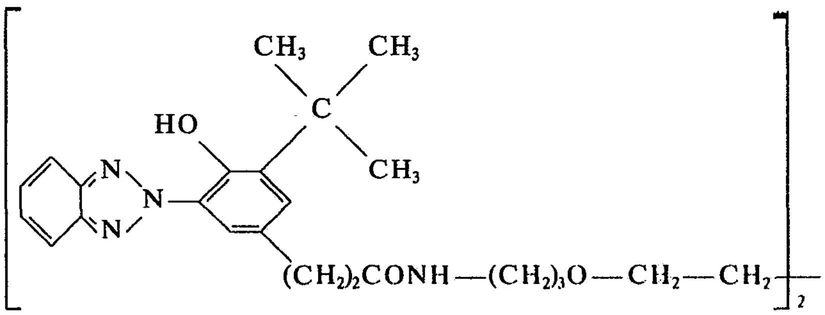

Smp. 158—159° C (Toluol) (Verb. 81)

mit 4,7,10-Trioxatetradecylamin

2-[2-Hydroxy-3-tert.butyl-5-(2-carbo-(4,7,10-trioxatetradecyl)-amidoäthyl)-phenyl]-benzotriazol. Smp.: 41—43° C (Hexan) (Verb. 82)

2-[2-Hydroxy-3-tert.butyl-5-(2-carbo-(4,7,10-trioxatetradecyl)-amidoäthyl)-phenyl]-5-chlorbenzotriazol. Smp.: 59—61° C (Hexan) (Verb. 83)

mit Hexamethylendiamin

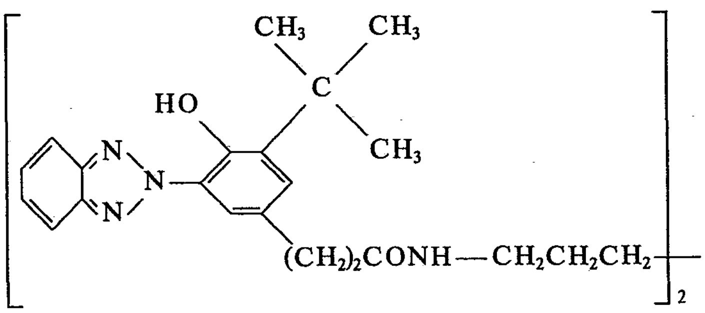

Smp.: 211—213° C (Toluol) (Verb. 84)

mit Ammoniak-Einleitung

2-[2-Hydroxy-3-tert.butyl-5-(2-carbamidoäthyl)-phenyl]-benzotriazol. Smp.: 159—160° C (Acetonitril) (Verb. 85)

## Beispiel 10

In einem 1-l-Autoklaven werden 33,9 g 2-[2-Hydroxy-3-tert.butyl-5-(2-carboxyäthyl)-phenyl]-benzotriazol in 300 ml Toluol suspendiert, mit 6,6 g (50% Überschuß zum Moläquivalent) Äthylenoxid versetzt und während 12 Stunden auf 80° C Innentemperatur erhitzt. Danach kühlt man ab und wäscht die Toluollösung mit verdünnter HCl-Lösung und nochmals mit verdünnter Kaliumcarbonatlösung. Die organische Phase wird getrocknet und eingedampft und der Rückstand umkristallisiert. Man erhält

2-[2-Hydroxy-3-tert.butyl-5-(2-carbo-(2-hydroxyäthyl-)phenyl]-benzotriazol. Smp.: 101—102° C (Toluol) (Verb. 86)

Mit der »Chlorbenzotriazolcarbonsäure« erhält man

2-[2-Hydroxy-3-tert.butyl-5-(2-carbo-(2-hydroxyäthyl-)phenyl]-5-chlorbenzotriazol. Smp.: 120—122° C (Logroin) (Verb. 87)

Verwendet man 0,1 Mol »Benzotriazolcarbonsäure« und 0,05 Mol Äthylenoxid und erhitzt 10 Stunden auf 150° C Innentemperatur, so erhält man folgende Verbindungen

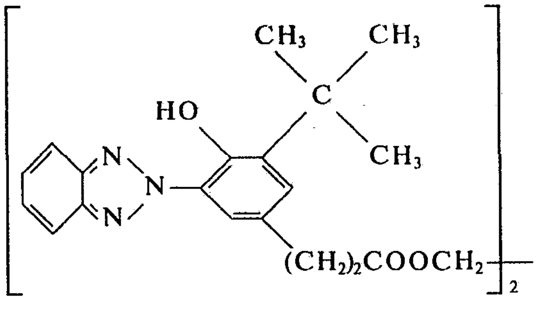

Smp.: 177—178° C (Toluol) (Verb. 88)

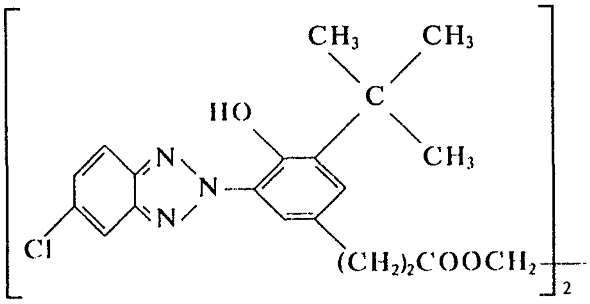

Smp.: 171—173° C (Toluol) (Verb. 89)

Beispiel 11

10,8 g 2-[2-Hydroxy-3-tert.butyl-5-(2-carbo-(2-hydroxyäthyloxy)äthyl)-phenyl]-benzotriazol (Verbindung aus Beispiel 10) werden in 100 ml Acetanhydrid auf ca. 80° C erhitzt. Man rührt die gelb-bräunliche Lösung ca. 15 Stunden, kühlt danach ab und trägt die Lösung auf Wasser aus. Man extrahiert mit Toluol und wäscht die Toluolphase noch mit verdünnter Kaliumcarbonatlösung und Wasser. Darauf trocknet man die Toluollösung und dampft ein. Der Rückstand wird aus Hexan umkristallisiert. Man erhält

2-[2-Hydroxy-3-tert.buryl-5-(2-carbo-(2-acetoxyäthyloxy)-äthyl)-phenyl]-benzotriazol.
Smp.: 92—93° C (Verb. 90)

Beispiel 12

77,6 g 2-[2-Hydroxy-3-tert.butyl-5-(2-carbomethoxyäthyl)-phenyl]-5-chlorbenzotriazol (Verbindung 6) werden in 300 ml Toluol p.a. vorgelegt. Dazu gibt man 13,0 g n-Octanol und 15,7 g 2-Äthylhexanol und erwärmt die Lösung auf Rückfluß. Bei 60°C gibt man 4,6 g (±)-Campher-10-sulfonsäure($\beta$) hinzu. Man destilliert nun während ca. 8 Stunden langsam ein Gemisch von Methanol/Toluol ab und ersetzt von Zeit zu Zeit die abdestillierte Menge durch frisches Toluol. Das DC zeigt nach ca. 8 Stunden Reaktionszeit praktisch einen quantitativen Umsatz. Man läßt die gelbe Lösung abkühlen und wäscht mit ca. 1 Liter warmem Wasser, danach mit ca. 1 Liter 5%iger Kaliumcarbonatlösung und nochmals mit Wasser. Man trennt die organische Schicht ab und trocknet sie über Natriumsulfat sicc. Die Toluollösung wird nun noch mit ca. 5 g Tonsil AC gereinigt, filtriert und eingedampft. Der Rückstand wird am Hochvakuum bei 0,04 Torr getrocknet. Man erhält 89,7 g (92,3%) eines hellgelben Öls, das ein Gemisch darstellt, bestehend aus

ca. 50% 2-[2-Hydroxy-3-tert.butyl-5-(2-carbo-(2-äthylhexyloxy)-äthyl)-phenyl]-5-chlorbenzotriazol (Verbindung 22) und

ca. 50% 2-[2-Hydroxy-3-tert.butyl-5-(2-carbooctyloxyäthyl)-phenyl]-5-chlorbenzotriazol (Verbindung 21)

Das gelbliche Öl wird durch NMR-Spektroskopie und Gaschromatographie analysiert.
Die Elementaranalyse ergibt folgende Werte:

| | | | | |
|---|---|---|---|---|
| Ber.: | C=66,72% | H=7,46% | N=8,64% | Cl=7,29% |
| Gef.: | C=66,84% | H=7,73% | N=8,60% | Cl=7,45% |

Verwendet man anstelle des Chlorbenzotriazols (Verbindung 6) den nicht-chlorierten Benzotriazolcarbonsäuremethylester (Verbindung 5) und verfährt im übrigen genau gleich, wie im obigen Beispiel beschrieben, so erhält man als Produkt wieder ein leicht gelbliches Öl, das ebenfalls ein (1 : 1)-Gemisch darstellt bestehend aus

2-[2-Hydroxy-3-tert.butyl-5-(2-carbooctyloxyäthyl)-phenyl]-benzotriazol (Verbindung 8) und

2-[2-Hydroxy-3-tert.butyl-5-(2-carbo-(2-äthylhexyloxy)-äthyl)-phenyl]-benzotriazol (Verbindung 9)

EA.:

| | | | |
|---|---|---|---|
| Ber.: | C=71,80% | H=8,25% | N=9,31% |
| Gef.: | C=72,17% | H=8,31% | N=9,23% |

Verwendet man anstelle von n-Octanol und 2-Äthyl-hexanol ein Gemisch bestehend aus Diäthylenglykolmonoethyläther und Diäthylenglykol-monobutyläther und verfährt im übrigen genau gleich wie

in Beispiel 12 beschrieben, so erhält man, ausgehend vom Benzotriazolcarbonsäuremethylester (Verbindung 5), wiederum ein leicht gelbliches Öl das ein (1 : 1)-Gemisch darstellt bestehend aus

2-[2-Hydroxy-3-tert.butyl-5-(2-carbo-(3,6-dioxaoctyloxy)-äthyl)-phenyl]-benzoriazol (Verbindung 63) und

2-[2-Hydroxy-3-tert.butyl-5-(2-carbo-(3,6-dioxadecyloxy)-äthyl)-phenyl]-benzotriazol (Verbindung 65)

EA für (1 : 1)-Gemisch:

| | | | |
|---|---|---|---|
| Ber.: | C=66,50% | H=7,51% | N=8,95% |
| Gef.: | C=66,50% | H=7,85% | N=8,90% |

Geht man vom Chlorbenzotriazolsäuremethylester (Verbindung 6) aus, so erhält man wieder ein (1 : 1)-Gemisch eines gelblichen Öls bestehend aus

2-[2-Hydroxy-3-tert.butyl-5-(2-carbo-(3,6-dioxaoctyloxy)-äthyl)-phenyl]-5-chlorbenzotriazol (Verbindung 64) und

2-[2-Hydroxy-3-tert.butyl-5-(2-carbo-(3,6-dioxadecyloxy)-äthyl)-phenyl]-5-chlorobenzotriazol (Verbindung 66)

EA für (1 : 1)-Gemisch:

| | | | | |
|---|---|---|---|---|
| Ber.: | C=61,96% | H=6,80% | N=8,34% | Cl=7,03% |
| Gef.: | C=62,21% | H=6,70% | N=8,36% | Cl=7,05% |

## Anwendungsbeispiele

### Beispiel I

Die Verbindungen 17, 18 und 21 werden in folgender Weise auf ihre Kristallisationsneigung geprüft.

Es werden Lösungen von jeweils 0,5 mMol dieser UV-Absorber und verschiedenen Mengen Trikresylphosphat (TKP) und Dibutylphthalat (DBP) in jeweils 20 ml Methylenchlorid hergestellt, diese ca. 5 Stunden bei 40°C erwärmt, um den größten Teil des Lösungsmittels zu entfernen und die Proben anschließend 15 Stunden bei 20°C im Hochvakuum belassen. Hierauf werden die Proben bezüglich Kristallisation des UV-Absorbers beurteilt. Die Resultate sind in Tabelle I zusammengestellt.

Tabelle 1

| | mg Produkt | 100 mg TKP | 200 mg TKP | 100 mg DBP | 200 mg DBP |
|---|---|---|---|---|---|
| Verbindung 21 | 241 | + | + | + | + |
| Verbindung 18 | 238 | + | + | + | + |
| Verbindung 17 | 224 | + | + | + | + |

\+ = klare Lösung
0 = Kristallisiert

### Beispiel II

Es werden 3 photographische Materialien aus einem polyäthylenbeschichteten, opaken Träger, einer darauf aufgebrachten lichtempfindlichen Schicht (Gelatine-Basis), die Silberbromid und einen Kuppler enthält und einer Deckschicht (Gelatine-Basis), die jeweils einen der folgenden 3 erfindungsgemäßen UV-Absorber enthält, hergestellt: Verbindung 17, 18 oder 21.

Es werden folgende Flächenkonzentrationen (Angaben für 1 $m^2$) erhalten.

Tabelle 2

| Komponente | AgBr-Schicht | Deckschicht |
|---|---|---|
| Gelatine | 5,6 g | 7 g |
| Härtungsmittel A | — | 430 mg |
| Netzmittel (anionisch) | 430 mg | 1100 mg |
| Silberbromid | 520 mg | — |
| Trikresylphosphat (TKP) | 1060 mg | 0/2500 mg |
| Magentakuppler | 207 mg | — |
| UV-Absorber | — | 1,1 mMol |

Kuppler und Härtungsmittel haben die folgende Konstitution:

Magentakuppler

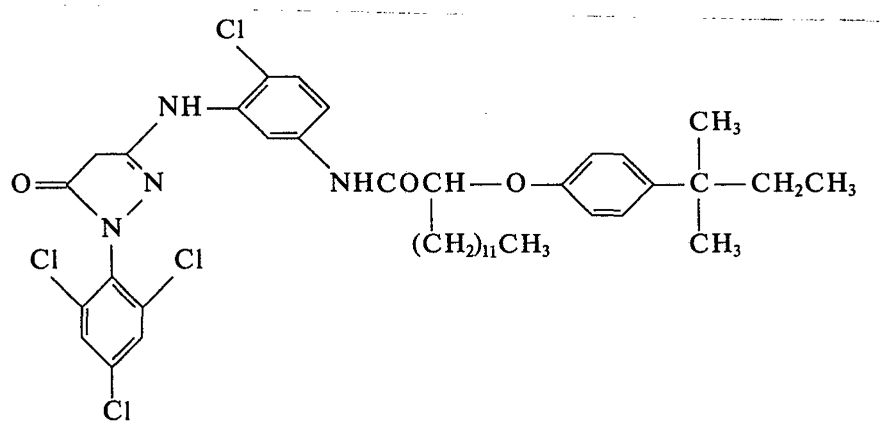

Härtungsmittel A

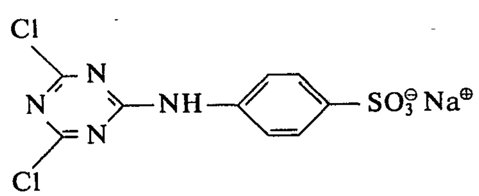

Auf das erhaltene Material wird jeweils ein Stufenkeil mit einem Dichteunterschied von 0,15 logE pro Stufe aufbelichtet und anschließend gemäß den Vorschriften des Herstellers im Verarbeitungsprozeß E+2 der Firma Kodak für Negativ-Farbpapiere verarbeitet.

Anschließend wird die Remissionsdichte im Grün für die Magentastufe mit einer Dichte zwischen 0,9 und 1,1 des Keils gemessen. Dann wird der Keil in einem Tageslicht-Belichtungsgerät mit total 10 000 Langley (41,9 KWs/cm²) belichtet und erneut die Remissionsdichte gemessen.

Aufgrund der so erhaltenen Werte lassen sich die in der Tabelle 3 aufgeführten prozentualen Dichteverluste berechnen.

Tabelle 3

| UV-Absorber | Dichteverlust nach 10 000 L | TKP |
|---|---|---|
| Nullprobe | 84% | ja |
| Verbindung 21 | 42% | nein |
| Verbindung 18 | 48% | ja |
| Verbindung 17 | 46% | ja |

Daraus ist ersichtlich, daß die erfindungsgemäßen UV-Absorber denjenigen des Standes der Technik bezüglich Schutzwirkung mindestens ebenbürtig sind und sie teilweise sogar übertreffen.

Beispiel III

Die Mischung der erfindungsgemäßen UV-Absorber in deckenden Klarlacken

Es werden folgende Lacke zugrunde gelegt:

a) Basislack

| | |
|---|---|
| Dynapol H 700®; Dynamit Nobel A.G. (Polyester) | 27,70Gew.-T. |
| Gemisch von<br>21% Zirkonyl-di-isooctoat<br>4% Zirkonyl-mono-isooctoat<br>75% Mineralöl (Sdpkt. 150—190° C) | 1,2 Gew.-T. |
| CAB-EAB 531.1®; Eastman Kodak<br>(Celluloseacetat/Butyrat, 25% in Xylol/Butylacetat (1 : 2)) | 17,00 Gew.-T. |
| Maprenal MF 650®; Hoechst A.G.<br>(Melaminharz, 50% in Butanol) | 2,95 Gew.-T. |
| Solvesso 150®; Shell (Lösungsmittelgemisch) | 10,55 Gew.-T. |
| Butylacetat | 21,10 Gew.-T. |
| Xylol | 10,50 Gew.-T. |
| Sparkle Silver 5400® (Al-Pigment) | 9,00 Gew.-T. |
| | 100,00 Gew.-T. |

b) Klarlack

| | |
|---|---|
| Viacryl VC 373®; Vianova A.G. (Acrylharz) | 58,30 Gew.-T. |
| Maprenal MF 590®; Hoechst A.G. | 27,30 Gew.-T. |
| Silikonöl, 1% in Xylol | 1,00 Gew.-T. |
| Solvesso 150® | 4,00 Gew.-T. |
| Xylol | 5,40 Gew.-T. |
| Äthylglycolacetat | 4,00 Gew.-T. |
| | 100,00 Gew.-T. |

Die erfindungsgemäßen Verbindungen werden im Spritzverdünner (Xylol/Butylacetat/Äthylglykolacetat, 1 : 1 : 1) vorgelöst und dem Klarlack zugemischt.

Die Lackierungen von Metallblechen efolgen durch Spritzauftrag. Die Schichtstärken betragen:

| | |
|---|---|
| Basislack | ca. 15 μ |
| Klarlack | ca. 40 μ. |

Als Kriterium für die Wirksamkeit wird die Bewitterungsstabilität des Decklackes der 2-Schicht Metalleffektlackierung verwendet. Die Stabilisierungswirkung drückt sich in längeren Bewitterungszeiten bis zum Eintritt von Rißbildung und starkem Glanzabfall verglichen mit der Kontrollprobe aus.

Die Stabilisierungswirkung wird mittels des Schnellbewitterungsgerätes UVCON® (Firma Atlas Electr. Dev. Comp., Chicago, Illinois, USA) bestimmt; Zyklus: 4 Std. UV, 60° C; 4 Std. Kondensation

50° C. Messungen: 20° C Glanz nach DIN 67 540; visuelle Beurteilung der Rißbildung. Die Versuchsergebnisse sind in Tabelle 4 zusammengestellt.

Tabelle 4

| Verbindung Nr. | 0,2% an der jeweiligen Verbindung (Std.) $t_{G/2}$ | (Std.) $t_R$ | 0,7% an der jeweiligen Verbindung (Std.) $t_{G/2}$ | (Std. $t_R$ |
|---|---|---|---|---|
| KONTROLLE | 800 | 2600 | 800 | 2600 |
| 16 | 1800 | 3800 | 1500 | 3400 |
| 20 | 1500 | 3600 | 1400 | 2600 |
| 19 | 1500 | 3600 | 1200 | 2600 |
| 17 | 1500 | 3400 | 1700 | 2600 |
| 15 | 1500 | 3600 | 2700 | 3600 |
| 51 | 1900 | 3600 | 1700 | 3400 |
| 33 | 1100 | 3400 | 1300 | 3200 |
| 67 | 1000 | 2600 | 1500 | 3400 |
| 72 | 900 | 2600 | 2200 | >3800*) |
| 73 | 1700 | 3600 | 1900 | 3600 |
| 65 | 1200 | 2600 | 1400 | 2600 |
| 76 | 1400 | 2600 | 1300 | 2600 |
| 80 | 1400 | 2600 | 2300 | >3800**) |
| 67 | 1100 | 2600 | 1900 | 3600 |

*) Stark matt nach 3800 Std.
**) stark matt nach 3500 Std.
$t_{G/2}$ Zeit bis zu 50% Glanzverlust
$t_R$ Zeit bis Rißbildung

Beispiel IV

Einbau der Benztriazolcarbonsäure bei der Synthese von Alkydharzen

a) Es wird ein Alkydharz in Anlehnung an eine Vorschrift der Firma Shell (DX 49) in folgender Weise und unter Verwendung eines mit Rührwerk, Inertgaseinleitung und Rückflußkühler mit Wasserabscheider ausgestatteten 2-l-Glasbehälters 2stufig hergestellt.

Verwendete Grundsubstanzen inMengen:

| | |
|---|---|
| Pthalsäureanhydrid | 461,0 g |
| Dedico Ricinenfettsäuren | 175,5 g |
| Cardura E*) | 381,5 g |
| Trimethyloläthan | 182,0 g |
| Xylol | 60 ml |

*) Cardura E ist ein Glycidester einer Monocarbonsäure (siehe Shell-Merkblatt für Kunstharze vom 6. 10. 74).

Die Herstellung erfolgt im zeitlichen Ablauf wie folgt:

| Zeit | Temperatur | Säurezahl mg KOH/g | Wasserabscheidung | |
|---|---|---|---|---|
| 1. Stufe | | | | |
| 0 | 23°C | | | Cardura E und Fettsäure eingefüllt; Heizung, Rührer und Stickstoff an. |
| 5 Min. | | | | Phthalsäureanhydrid hinzugefügt. |
| 30 Min. | 150°C | | | Heizung bei 140°C abgestellt. Exotherm. Heizung falls erforderlich zur Beibehaltung der Temperatur von 150°C. |
| 1 Std. 15 Min. | 150°C | | | WPE* 56,000. Heizung abgestellt, Kühlung angestellt. |
| 2. Stufe | | | | |
| 1 Std. 35 Min. | 120°C | 120 | | Trimethyloläthan, Benztriazolcarbonsäure in Konzentrationen von 2 oder 4 oder 6% (bez. auf Gesamtgemisch) und Xylol hinzugefügt. Heizung angestellt. |
| 2 Std. 40 Min. | 180°C | | | |
| 3 Std. | 200°C | | die ersten Tropfen | |
| 3 Std. 30 Min. | 240°C | | | Reaktionstemperatur erreicht. |
| 4 Std. | 240°C | 26,8 | 30 ml | |
| 4 Std. 30 Min. | 240°C | 18,5 | 32 ml | |
| 5 Std. | 240°C | 14,5 | 34 ml | |
| 5 Std. 15 Min. | | | | Heizung abgestellt, kühlen und verdünnen. |

*) WPD = Epoxydäquivalentgewicht

Die Reaktanten der esten Stufe, Cardura E, Ricinenfettsäure und Phthalsäureanhydrid werden in den Kessel gefüllt, und die Temperatur wird unter lansamer Stickstoffeinleitung auf 150°C gebracht. Die Temperatur wird dann auf 150°C gehalten, bis ein Epoxidäquivalentgewicht von 50—70,000 erreicht ist (3/4—1 Std.). Der Ansatz wird dann bis auf etwa 120°C abgekühlt.

Der Ansatz wird nach Erreichen folgender Säurezahlen abgestoppt.

| Benztriazolcarbonsäure (Gew.-%) | Säurezahl (mg/KOH/g) |
|---|---|
| 2% | 14,5 |
| 4% | 16,5 |
| 6% | 16,8 |

Die abgekühlte Alkydharzlösung wird mit Xylol verdünnt und in einer Trockenfilmstärke von ca. 10 μ auf Quarzplatten aufgezogen.

Zur Überprüfung des Einbaus werden diese Proben während 10 Tagen bei 100° C warmgelagert und aus den UV-Transmissionswerten vor und nach Warmlagerung die Lichtschutzmittelverluste berechnet.

Tabelle 5

| Benztriazolcarbonsäure (Gew.-%) | Verluste nach 10 Tagen Warmlagerung (Gew.-%) |
|---|---|
| 2 | <5% |
| 4 | <5% |
| 6 | <5% |
| 4% (nicht einkondensiert, zum Vergleich) | 100% |

b) Es wird ähnlich wie unter a) beschrieben ein Alkydharz hergestellt (Shell Vorschrift DX 51).

1. Stufe:

| | |
|---|---|
| Phthalsäureanhydrid | 569,4 g |
| Glycerin | 23,4 g |
| Cardura E | 612,7 g |

2. Stufe:

| | |
|---|---|
| Glycerin | 94,5 g |
| Xylol | 60 ml |

Zeitlicher Ablauf der Herstellung:

| Zeit | Temperatur | Säurezahl mg/KOH/g | Wasserabscheidung | |
|---|---|---|---|---|
| 1. Stufe | | | | |
| 0 | 23°C | | | Cardura E, Phthalsäureanhyrid und Glycerinmenge der erstenStufe eingefüllt. Heizung, Rührer und Stickstoff angestellt. |
| 40 Min. | 150°C | | | Heizung abgestellt. |
| 1 Std. 10 Min. | 150°C | | | 130—140°C. Exotherm. Heizung bis zu 180°C |
| 1 Std. 20 Min. | 180°C | | | |
| 1 Std. 30 Min. | 180°C | | | Heizung abgestellt, Epoxidäquivalentgewicht 66,000, Kühlung |
| 2. Stufe | | | | |
| 1 Std. 40 Min. | 140°C | | | Glycerinmenge der zweiten Stufe, Benztriazolcarbonsäure in Konzentrationen von 2 oder 4 oder 6% und Xylol hinzugefügt. |
| 2 Std. 40 Min. | 200°C | | die ersten Tropfen | Rückfluß |
| 3 Std. 30 Min. | 240°C | | | |
| 4 Std. | 240°C | 16,9 | 18 ml | |
| 4 Std. 30 Min. | 240°C | 14,5 | 20 ml | |
| 5 Std. | 240°C | 12,2 | 22 ml | |
| 5 Std. 15 Min. | 240°C | | 24 ml | Heizung abgestellt, kühlen und verdünnen |

| | |
|---|---|
| Festkörpergehalt | 70 Gew.-% in Xylol |
| Viskosität | 48 P bei 25°C |
| Säurezahl | 11,4 mg KOH/g |

Die Reaktionsstoffe der ersten Stufe Cardura E, Phthalsäureanhyrid und Glycerin werden in den Kessel gefüllt, und die Temperatur wird bei langsamer Stickstoffeinleitung auf 150°C gebracht. Die Temperatur wird für 1/2 Stunde gehalten, dann auf 180°C gebracht und etwa 15—30 Minuten lang auf dieser Höhe gehalten, bis die Epoxidäquivalentgewichts-Messung einen Wert von 50 000 bis 70 000 zeigt und dann auf 130—140°C abgekühlt.

Die Glycerinmenge der zweiten Stufe wird dann zusammen mit der Benztriazolcarbonsäure und Xylol zugefügt, und die Temperatur wird über einen Zeitraum von einerStunde auf 190—200°C gebracht.

Die Temperatur wird unter Beibehaltung eines ständigen Rückflusses auf 230—240°C erhöht und bis zu einer endgültigen Säurezahl von 13—17 mg KOH/g gehalten. Der Ansatz wird dann abgekühlt und mit Xylol auf 70% Festkörpergehalt verdünnt.

Erreichte Säurezahlen:

| Benztriazolcarbonsäure (Gew.-%) | Säurezahl (mg KOH/g) |
|---|---|
| 2% | 17,1 |
| 4% | 13,0 |
| 6% | 13,8 |

Zur Überprüfung des Einbaus wird ebenfalls wie zuvor beschrieben vorgegangen.

Tabelle 6

| Benztriazolcarbonsäure (Gew.-%) | Verluste nach 10 Tagen Warmlagerung (Gew.-%) |
|---|---|
| 2 | <5 |
| 4 | <5 |
| 6 | <5 |

Beispiel V

a) Im folgenden Versuch werden außer dem Magentakuppler (Beispiel II) folgende Kuppler verwendet.

Gelbkuppler:

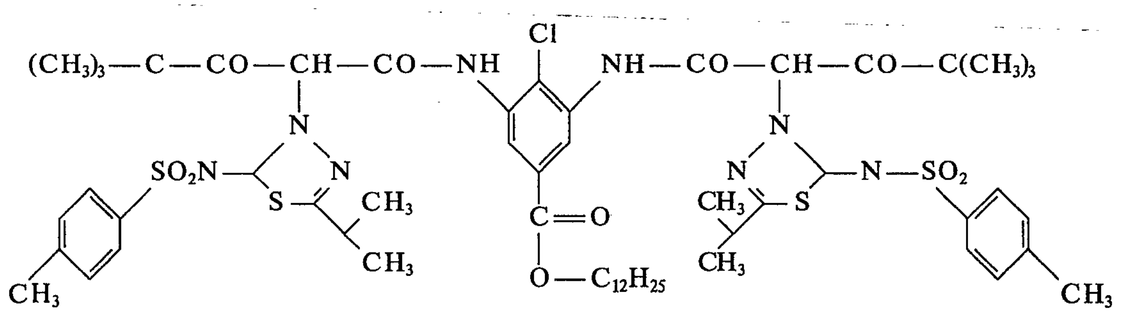

Cyankuppler:

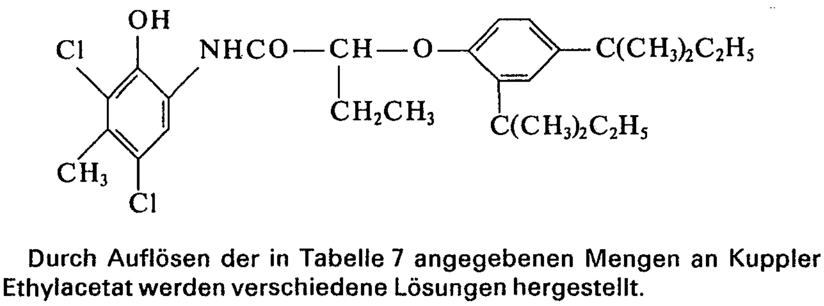

Durch Auflösen der in Tabelle 7 angegebenen Mengen an Kuppler und UV-Absorber in je 100 ml Ethylacetat werden verschiedene Lösungen hergestellt.

Tabelle 7

| Lösung Nr. | Kuppler | Menge (mg) | Verbindung Nr. | Menge (mg) |
|---|---|---|---|---|
| 1 | Gelbkuppler | 299 | L*) | 226+226 |
| 2 | Gelbkuppler | 299 | L | 151+151 |
| 3 | Gelbkuppler | 299 | 62 | 499 |
| 4 | Gelbkuppler | 299 | 62 | 333 |
| 5 | Gelbkuppler | 299 | Trikresyl-phosphat | 300 |
| 6 | Magentakuppler | 194 | L | 226+226 |
| 7 | Magentakuppler | 194 | L | 151+151 |
| 8 | Magentakuppler | 194 | 62 | 499 |
| 9 | Magentakuppler | 194 | 62 | 333 |
| 10 | Magentakuppler | 194 | Trikresyl-phosphat | 300 |
| 11 | Cyankuppler | 124 | L | 226+226 |
| 12 | Cyankuppler | 124 | L | 151+151 |
| 13 | Cyankuppler | 124 | 62 | 499 |
| 14 | Cyankuppler | 124 | 62 | 333 |
| 15 | Cyankuppler | 124 | Trikresyl-phosphat | 300 |

*) L ist eine 1 : 1-Mischung von Verbindung 63 und 65.

Die Lösungen 5, 10 und 15 enthalten anstelle des UV-Absorbers Trikresylphosphat als Lösungsmittel für den Kuppler (Null-Probe).

Je 2,0 ml dieser Lösungen werden zu je einer Mischung von 7,0 ml einer 6%igen Gelatinelösung (pH 6,5), 0,4 ml einer 8%igen Lösung von Triton 770® (anionisches Netzmittel der Firma Rohm und Haas) in Wasser und 0,6 ml Wasser gegeben und das Gemisch mit Hilfe von Ultraschall (5 Minuten, 100 Watt) emulgiert.

Zu je 2,5 ml der so erhaltenen Emulsionen werden je 2,8 ml Wasser, 0,7 ml einer 1%igen Lösung des Härters der Formel

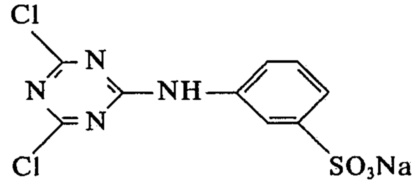

und 2,0 ml einer Silberbromid-Emulsion (7 g Silber pro kg Emulsion) zugesetzt. Dann wird das Gemisch auf einem auf einer Glasplatte aufgezogenen, kunststoffbeschichteten Papier von 13 x 18 cm gleichmäßig verteilt.

Nach einer Trocknungszeit von 7 Tagen bei Raumtemperatur werden je 2 Stufenkeile aufbelichtet und im Ektaprint+2-Prozeß der Firma Kodak gemäß Angaben des Herstellers verarbeitet. Dann werden die so erhaltenen Proben wie folgt behandelt:

Lichtechtheit:
Bestrahlung in einem Atlas Weather Ometer mit einer Xenonlampe mit der in Tabelle 8 angegebenen Energiemenge bei 43° C und 55% relativer Luftfeuchtigkeit.

Dunkelstabilität:
Die Proben mit dem Cyankuppler werden während 28 Tagen bei 60° C und 70% relativer Luftfeuchtigkeit im Dunkeln gelagert.

Tabelle 8 enthält die Dichteverluste in Prozent bei einer ursprünglichen Dichte von 1.0 (Remission).

Tabelle 8

| Probe Nr.*) | Lichtechtheit Bestrahlungsenergie ($kJ/cm^2$) | Dichteverlust (%) | Dunkelstabilität Dichteverlust (%) |
|---|---|---|---|
| 1 | 21 | 23 | |
| 2 | 21 | 21 | |
| 3 | 21 | 25 | |
| 4 | 21 | 25 | |
| 5 (Referenz) | 21 | 40 | |
| 6 | 42 | 50 | |
| 7 | 42 | 56 | |
| 8 | 42 | 75 | |
| 9 | 42 | 77 | |
| 10 (Referenz) | 42 | 80 | |
| 11 | 63 | 21 | 13 |
| 12 | 63 | 24 | 17 |
| 13 | 63 | 22 | 8 |
| 14 | 63 | 25 | 6 |
| 15 (Referenz) | 63 | 27 | 25 |

*) Die Probe-Nr. basiert jeweils auf der Lösung Nr. aus Tabelle 7.

Aus Tabelle 8 geht hervor, daß die Verbindungen gemäß der vorliegenden Erfindung auch als Lösungsmittel für öllösliche chromogene Kuppler eingesetzt werden können. Hierbei wird im Falle des Gelbkupplers die Lichtechtheit, im Falle des Cyankupplers die Dunkelstabilität deutlich verbessert.

b) Zur Verhinderung der Diffusion von oxydierter Entwicklersubstanz in benachbarte kupplerhaltige Schichten und damit zur Vebesserung der Farbwiedergabe enthalten Farbmaterialien in der Regel sogenannte Zwischenschichten, die ihrerseits oft noch eine diffusionsfest eingelagerte Vebindung enthalten, die mit dem oxydierten Entwickler zu farblosen Produkten zu reagieren vermag, wodurch die Farbtrennung noch weiter verbessert wird.

Das vorliegende Beispiel zeigt, daß sich die erfindungsgemäßen Substanzen auch als Lösungsmittel für solche Verbindungen eignen.

Auf einem transparenten Träger wird eine Materialprobe A folgender Zusammensetzung hergestellt (Herstellung der Dispersion analog zu Beispiel II):

1. Lichtempfindliche Schicht

Die Schicht enthält als Magentakuppler die Verbindung aus Beispiel II in einer Flächenkonzentration von 450 mg/m², gelöst in Trikresylphosphat (Flächenkonzentration 180 mg/m²), Silberbromid in einer Flächenkonzentration von 500 mg Silber pro m², den Härter aus Beispiel II (Flächenkonzentration 69 mg/m²) und Gelatine. Die Schichtdicke beträgt 3,7 µm.

2. Zwischenschicht

Die Schicht enthält eine Dispersion von 2,5-Di-tert.-octyl-hydrochinon (Flächenkonzentration 300 mg/m²), gelöst in 600 mg eines 50 : 50-Gemisches der erfindungsgemäßen Verbindungen Nr. 21 und 22 den Härter aus Beispiel II (Flächenkonzentration 48 mg/m²) und Gelatine. Die Schichtdicke beträgt 3,0 µm.

3. Kupplerhaltige Nachbarschicht

Die Schicht enthält eine Dispersion des Cyankupplers aus Beispiel V (Flächenkonzentration 400 mg/m²) in Trikresylphosphat (Flächenkonzentration 200 mg/m²), den Härter aus Beispiel II (Flächenkonzentration 36 mg/m²) und Gelatine. Die Schichtdicke beträgt 2,2 µm.

4. Deckschicht

Die Deckschicht enthält Gelatine und den Härter aus Beispiel II (Flächenkonzentration 35 mg/m²). Die Schichtdicke beträgt 1,5 µm.

Im Weiteren wurden die folgenden Proben hergestellt:

Probe B:
Wie Probe A, aber nur Gelatine in der Zwischenschicht

Probe C:
Wie Probe A, aber Cyankuppler in der kupplerhaltigen Nachbarschicht ersetzt durch den Gelbkuppler aus Beispiel Va (Flächenkonzentration 500 mg/m²)

Probe D:
Wie Probe C, aber nur Gelatine in der Zwischenschicht.

Die Proben werden total belichtet und wie unter a) angegeben verarbeitet.

Anschließend werden die optischen Dichten bei 446 nm (Absorptionsmaximum des Gelbfarbstoffes), 535 nm (Absorptionsmaximum des Magentafarbstoffes) und 662 nm (Absorptionsmaximum des Cyanfarbstoffes) gemessen. Die entsprechenden Werte sind in der Tabelle 9 enthalten.

Tabelle 9

| Probe | $D_{446nm}$ | $D_{535nm}$ | $D_{662nm}$ |
|---|---|---|---|
| A | 0,11 | 0,80 | 0,04 |
| B | 0,11 | 0,82 | 0,08 |
| C | 0,12 | 0,82 | 0,03 |
| D | 0,16 | 0,83 | 0,03 |

Aus den Daten in der Tabelle 9 geht eindeutig hervor, daß durch die Einlagerung eines Gemisches aus 2,5-Di-tert.octyl-hydrochinon und der erfindunsgemäßen Verbindungen Nr. 21 und 22 die Bildung von Cyan-, resp. Gelbfarbstoff in einer benachbarten Schicht deutlich unterdrückt wird.

Die Proben werden anschließend in einem Weather Ometer derFirma Atlas mit total 21 kJ/cm² bestrahlt. Die Dichteverluste bei 535 nm betragen:

Tabelle 10

| Proben | Dichteverlust in Prozent |
|---|---|
| A | 26 |
| B | 53 |
| C | 28 |
| D | 53 |

c) Das Beispiel zeigt, daß sich die Verbindungen der vorliegenden Erfindung auch als Lösungsmittel für andere UV-Absorber verwenden lassen, wobei je nach den Absorptionsspektren eine gewünschte spektrale Absorption erhalten werden kann.

Lösungen der Verbindungen Nr. 63 und Nr. 65 der vorliegenden Erfindung und 2-[2-Hydroxy-3(1-methyl-propyl)-5-tert.butyl-phenyl]-2-H-benztriazol (Vebindung Q) in Essigester werden wie in Beispiel II angegeben in einer wäßrigen Gelatine-Lösung dispergiert. Den so erhaltenen Emulsionen wird Härter des Beispiels II zugesetzt (7 mMol/100 g Gelatine) und die Gemische auf substrierte Glasplatten verteilt, wobei die in der Tabelle 11 aufgeführten Flächenkonzentrationen erhalten werden.

Die Flächenkonzentration an Gelatine beträgt in allen Fällen 4,8 g/m².

Tabelle 11

| Probe | Flächenkonzentrationen, mg/m² Verb. 63 | Verb. 65 | Verb. Q | $D_{max}$ | $\lambda_{max}$ | $D_{380nm}$[1]) | $D_{390nm}$[1]) | $D_{400nm}$[1]) |
|---|---|---|---|---|---|---|---|---|
| E | 300 | 300 | 123 | 2,15 | 350 | 1,02 | 0,47 | 0,14 |
| F | 225 | 225 | 246 | 2,30 | 349 | 1,03 | 0,46 | 0,14 |
| G | 150 | 150 | 370 | 2,41 | 348 | 0,98 | 0,41 | 0,14 |
| H | 75 | 75 | 490 | 2,54 | 346 | 0,84 | 0,28 | 0,08 |

[1]) opt. Dichte bei der entsprechenden Wellenlänge

d) Der Farbstoff der Formel

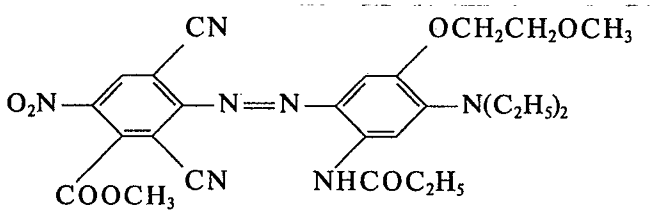

wird einmal in einem 1 : 1-Gemisch der erfindungsgemäßen Verbindungen Nr. 21 und Nr. 22 und einmal in Trikresylphosphat mit Ethylacetat als Hilfslösungsmittel gelöst und wie in Beispiel II angegeben dispergiert. Nach Zusatz einer Silberbromid-Emulsion wird die Dispersion wie in Beispiel Va angegeben auf kunststoffbeschichtetes Papier vergossen, wobei die folgenden Flächenkonzentrationen erhalten werden:

| Komponente | Probe I | Probe K (Vergleichsprobe) |
|---|---|---|
| Farbstoff | 118 mg/$m^2$ | 118 mg/$m^2$ |
| Verbindung Nr. 21 | 1,07 g/$m^2$ | — |
| Verbindung Nr. 22 | 1,07 g/$m^2$ | — |
| Trikresylphosphat | — | 2,4 g/$m^2$ |
| Silber | 507 mg/$m^2$ | 507 mg/$m^2$ |
| Gelatine | 8,3 g/$m^2$ | 8,3 g/$m^2$ |
| Härter (siehe unter a) | 490 mg/$m^2$ | 490 mg/$m^2$ |

Hinter einem Stufenkeil wird das so erhaltene lichtempfindliche Material belichtet und bei 24° C wie folgt verarbeitet:

| | |
|---|---|
| Entwicklung | 6 Minuten |
| Wässerung | 4 Minuten |
| Silber- und Farbbleichung | 6 Minuten |
| Wässerung | 2 Minuten |
| Fixierung | 8 Minuten |
| Wässerung | 6 Minuten |
| Trocknung | |

Entwickler- und Fixierbäder sind übliche Bäder, wie sie in der Schwarzweißphotographie verwendet werden. Das Silberfarbbleichbad besitzt pro Liter Lösung folgende Zusammensetzung:

| | |
|---|---|
| Sulfaminsäure | 100 g |
| m-Nitrobenzolsulfonsäure | 10 g |
| Kaliumjodid | 6 g |
| 2,3,6-Trimethylchinoxalin | 2 g |
| 4-Mercaptobuttersäure | 1 g |

Die so erhaltenen Farbkeile werden nach Ausmessen der Farbdichte in einem Atlas Weather Ometer, das mit einer Xenonlampe ausgerüstet ist, mit total 21 kJ/$cm^2$ bestrahlt und anschließend erneut ausgemessen. Ausgehend von einer Dichte von 1,0 findet man folgende Dichteabnahmen:

| | |
|---|---|
| Probe I | 0,31 |
| Probe K (Vergleichsprobe) | 0,44 |

Daraus ist ersichtlich, daß durch die Verwendung der erfindunsgemäßen Verbindungen als Lösungsmittel für Farbstoffe für das Silberfarbbleich-Verfahren die Lichtechtheit der betreffenden Farbstoffe erhöht werden kann.

**Patentansprüche**

1. Verbindungen der Formel I

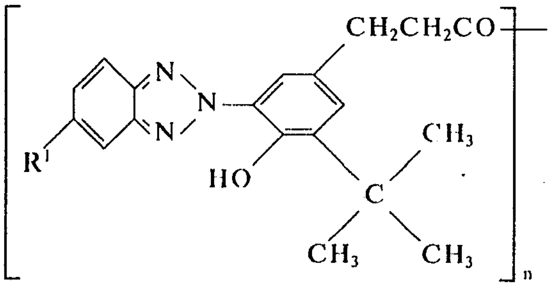

(I)

in der $R^1$ H, Cl, $C_1-C_4$-Alkyl oder $C_1-C_4$-Alkoxy und n eine der Zahlen 1 oder 2 bedeuten und in der $R^2$

a) im Falle von n = 1

Cl $-OR^3$ oder 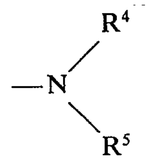

und

b) im Falle von n = 2 einen der zweiwertigen Reste

$-O-R^9-O-$ oder 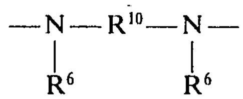

darstellt, wobei

$R^3$ H, gegebenenfalls durch 1 bis 3 OH-Gruppen oder durch

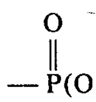 $-O-Si(R^6)_3$ oder $-O-CO(R^6)$

substituiertes $C_1-C_{18}$-Alkyl, durch $-O-$, $-S-$ oder $-NR^6-$ ein- oder mehrfach unterbrochenes $C_3-C_{18}$-Alkyl, das gegebenenfalls durch OH oder durch $-O-CO(R^6)$ substituiert sein kann, gegebenenfalls durch OH substituiertes $C_5-C_{12}$-Cycloalkyl, gegebenenfalls durch OH substituiertes $C_2-C_{18}$-Alkenyl, $C_7-C_{15}$-Aralkyl,

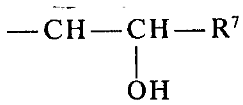 oder 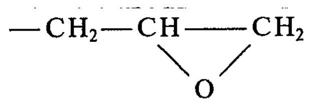

und

$R^4$ und $R^5$ unabhängig voneinander H, $C_1-C_{18}$-Alkyl, durch O, S oder $-NR^6-$ ein- oder mehrfach unterbrochenes $C_3-C_{18}$-Alkyl, $C_5-C_{12}$-Cycloalkyl, $C_6-C_{14}$-Aryl, $C_3-C_8$-Alkenyl, $C_7-C_{15}$-Aralkyl, $C_6-C_{14}$-Aryl oder $C_1-C_3$-Hydroxyalkyl bedeuten, oder aber $R^4$ und $R^5$ zusammen mit dem Stickstoffatom einen Pyrrolidin-, Piperidin-, Piperazin- oder Morpholinring darstellen,

und wobei

$R^6$ H, $C_1-C_{18}$-Alkyl, $C_5-C_{12}$-Cycloalkyl, $C_3-C_8$-Alkanyl, $C_6-C_{14}$-Aryl oder $C_7-C_{18}$-Aralkyl,

$R^7$ H, gegebenenfalls durch

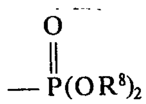

substituiertes $C_1-C_{18}$-Alkyl, gegebenenfalls durch OH substituiertes Phenyl, $C_7-C_{18}$-Aralkyl oder $-CH_2OR^8$,

$R^8$ $C_1-C_{18}$-Alkyl, $C_3-C_{18}$-Alkenyl, $C_5-C_{10}$-Cycloalkyl, $C_6-C_{16}$-Aryl oder $C_7-C_{15}$-Aralkyl,
$R^9$ $C_2-C_8$-Alkylen, $C_4-C_8$-Alkenylen, $C_4$-Alkinylen, Cyclohexylen, durch —O— ein- oder mehrfach unterbrochenes $C_4-C_{10}$-Alkylen,

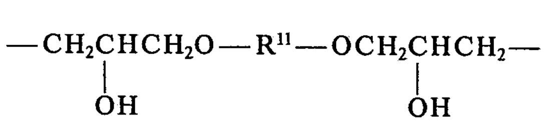 oder 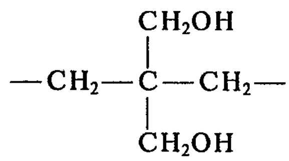

und
$R^{10}$ gegebenenfalls durch —O— ein- oder mehrfach unterbrochenes $C_2-C_{12}$-Alkylen, Cyclohexylen,

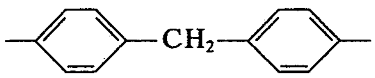 oder 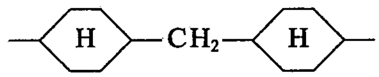

bedeuten.

oder aber $R^{10}$ und $R^6$ zusammen mit den beiden Stickstoffatomen einen Piperazinring darstellen, und wobei

$R^{11}$ $C_2-C_8$-Alkylen, durch —O— ein- oder mehrfach unterbrochenes $C_4-C_{10}$-Alkylen, 1,3- oder 1,4-Cyclohexylen, 1,3- oder 1,4-Phenylen,

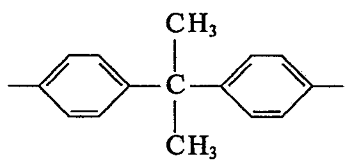 oder 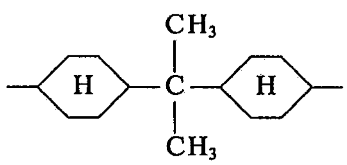

bedeutet.

2. Verbindungen nach Anspruch 1 der Formel I, in der $R^1$ H oder Cl, n die Zahl 1 und $R^2$

$-OR^3$ oder 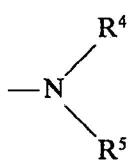

bedeuten, wobei

$R^3$ $C_4-C_{12}$-Alkyl, durch —O— unterbrochenes $C_3-C_{10}$-Alkyl, Cyclohexyl oder

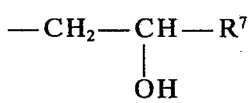

$R^4$ und $R^5$ unabhängig voneinander $C_4-C_{12}$-Alkyl, durch —O— unterbrochenes $C_3-C_{10}$-Alkyl oder Cyclohexyl,
$R^7$ gegebenenfalls durch

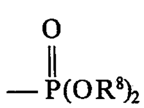

substituiertes $C_1-C_{12}$-Alkyl, oder $-CH_2OR^8$, und
$R^8$ $C_1-C_{12}$-Alkyl oder Cyclohexyl darstellen.

3. Verbindungen nach Anspruch 1 der Formel I, in der $R^1$ H oder Cl, n die Zahl 2, $R^2$ einen der zweiwertigen Reste

$-O-R^9-O-$ oder 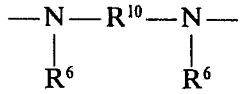

wobei

$R^6$ H oder $C_1-C_8$-Alkyl,
$R^9$ $C_2-C_6$-Alkylen, durch $-O-$ unterbrochenes $C_4-C_{10}$-Alkylen oder

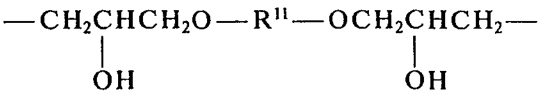

$R^{10}$ $C_2-C_{12}$-Alkylen, und
$R^{11}$ $C_2-C_6$-Alkylen oder durch $-O-$ unterbrochenes $C_4-C_{10}$-Alkylen

bedeuten.

4. Verbindung nach Anspruch 1, dadurch gekennzeichnet, daß es sich um

2-[2-Hydroxy-3-tert.butyl-5-(2-carbo-n-octyloxyäthyl)-phenyl]-benztriazol oder
2-{2-Hydroxy-3-tert.butyl-5-[2-carbo-(2-äthylhexyl)-oxyäthyl]-phenyl}-benztriazol oder
2-[2-Hydroxy-3-tert.butyl-5-(2-carbo-n-octyloxyäthyl)-phenyl]-5-chlorbenztriazol oder
2-{2-Hydroxy-3-tert.butyl-5-[2-carbo-(2-äthylhexyl)-oxyäthyl]-phenyl}-5-chlorbenztriazol oder
die Verbindung der Formel

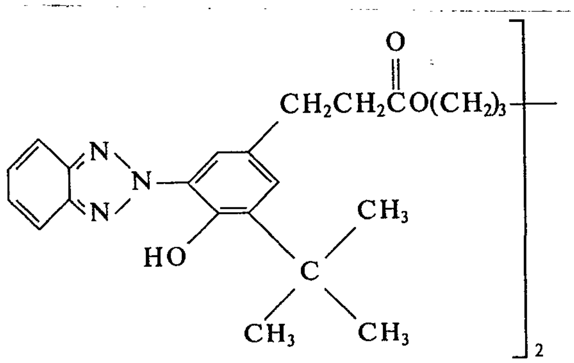

handelt.

5. Vebindung nach Anspruch 1, dadurch gekennzeichnet, daß ein Gemisch von

2-[2-Hydroxy-3-tert.butyl-5-(2-carbo-n-octyloxyäthyl)-phenyl]-5-chlorbenztriazol und
2-{2-Hydroxy-3-tert.butyl-5-[2-carbo-(2-äthylhexyl)-oxyäthyl]-phenyl}-5-chlorbenztriazol

im Gewichtsverhältnis 1 : 1 vorliegt.

6. Stabilisiertes organisches Material enthaltend in der Masse vorzugsweise 0,1 bis 5 Gew.-% einer Verbindung nach Anspruch 1.

7. Stabilisiertes Material nach Anspruch 6, dadurch gekennzeichnet, daß das Material ein synthetisches Polymer ist.

8. Stabilisiertes Polymer nach Anspruch 7, dadurch gekennzeichnet, daß das Polymer ein Polyester, heißvernetzbares Acrylharz, thermoplastisches Acrylharz, Polyamid, MF- oder UF-Harz, oder ein Polyurethan ist.

9. Stabilisierter Lack auf der Basis einer oder mehrerer Polymere gemäß Anspruch 8.

10. Stabilisiertes Material nach Anspruch 6, dadurch gekennzeichnet, daß es in Form von Photomaterial vorliegt oder Teile eines Photomaterials ist, wobei das Photomaterial, vorzugsweise in Deckschichten, 0,1 bis 5 Gew.-% einer Verbindung der Formel I enthält.

11. Verfahren zur Herstellung von Benztriazolcarbonsäuren, deren Estern und Säurechloriden der Formel I nach Anspruch 1 in der Weise, daß man ein gegebenenfalls substituiertes, diazotiertes o-Nitranilin der Formel II

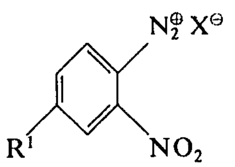

(II)

in der $X^{\ominus}$ ein Halogen-anion, $-HSO_4^{\ominus}$ oder $(BF_4)^{\ominus}$ bedeutet, mit 2-(3-tert.-Butyl-4-hydroxyphenyl)-propionsäure bei pH 8 bis 11 kuppelt und aus dem erhaltenen Farbstoff durch reduzierende Triazolierung unter alkalischen Bedingungen die entsprechende Benztriazolcarbonsäure herstellt,

und daß man gegebenenfalls danach die erhaltene Benztriazolsäure in Gegenwart eines sauren Katalysators entweder im Falle von n=1 in Formel I mit einem Alkohol der Formel $R^{3'}-OH$, wobei $R^{3'}$ die für Formel I angegebene Definition von $R^3$ hat, aber nicht

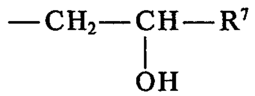

bedeutet, oder im Falle von n=2 in Formel I mit einem Alkohol der Formel $HO-R^9-OH$ direkt verestert,
oder daß man die Benztriazolsäure mit einer Epoxidverbindung der Formel

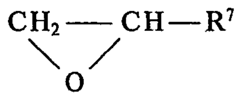

umsetzt,
oder daß man die Benztriazolsäure mittels Thionylchlorid im Überschuß in Kohlenwasserstoff-Suspension zum Säurechlorid chloriert.

12. Verfahren zur Herstellung von Benztriazolcarbonsäureestern der Formel I nach Anspruch 1 in der Weise, daß man einen gegebenenfalls nach dem Verfahren gemäß Anspruch 11 erhaltenen Benztriazolcarbonsäureester (vorzugsweise den Methyl- oder Äthylester) in Gegenwart von Umesterungskatalysatoren, wie Tetrabutylorthotitanat, Li-Amid oder Na-Methylat, im Falle von n=1 in Formel I mit Alkoholen der Formel $R^{3'}-OH$ oder im Falle von n=2 in Formel I mit Dialkoholen der Formel $HO-R^9-OH$ unter Abdestillieren des freigewordenen Alkohols umestert, wobei $R^3$ die in Anspruch 11 angegebene Bedeutung hat.

13. Verfahren zur Herstellung von Benztriazolcarbonsäureamiden der Formel I nach Anspruch 1 in der Weise, daß man einen gegebenenfalls nach dem Verfahren gemäß Anspruch 10 erhaltenen Benztriazolcarbonsäureester (vorzugsweise den Methyl- oder Äthylester) in Gegenwart von Katalysatoren, wie Li-Amid oder Na-Methylat, im Falle von n=1 in Formel 1 mit Ammoniak oder einem primären oder einem sekundären Amin der Formel

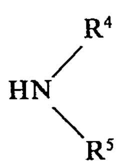

oder aber im Falle von n=2 in Formel I mit Diaminen der Formel

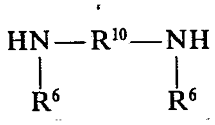

jeweils unter Abdestillation des frei werdenden Alkohols thermisch umsetzt.

**Claims**

1. A compound of the formula I

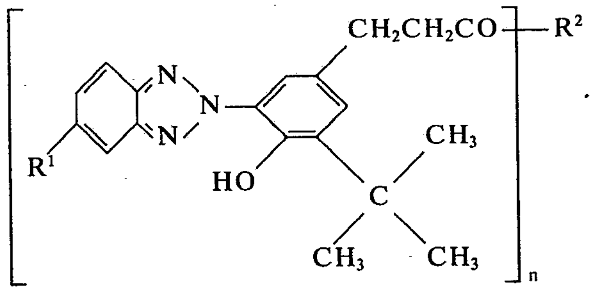

(I)

wherein $R^1$ is H, Cl, $C_1-C_4$ alkyl or $C_1-C_4$alkoxy and n is 1 or 2, and wherein

a) if n = 1, $R^2$ is

Cl $-OR^3$ or 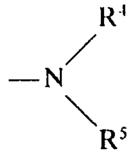

and

b) if n = 2, $R^2$ is one of the divalent radicals

$-O-R^9-O-$ or 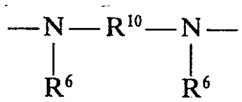

wherein

$R^3$ is H, straight-chain or branched $C_1-C_{18}$alkyl which is unsubstituted or substituted by 1 to 3 OH groups or by

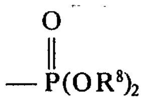 $-O-Si(R^6)_3$ or $-O-CO(R^6)$

or is $C_3-C_{18}$alkyl which is interrupted by one or more of $-O-$, $-S-$ or $-NR^6-$ and which can e unsubstituted or substituted by OH or $-O-CO(R^6)$, or is $C_5-C_{12}$cycloalkyl which is unsubstituted or substituted by OH, $C_2-C_{18}$alkenyl which is unsubstituted or substituted by OH, or is $C_7-C_{15}$aralkyl,

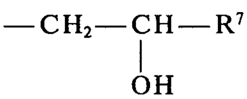 or 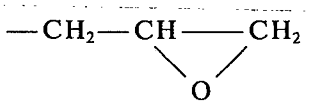

$R^4$ and $R^5$ are each independently H, $C_1-C_{18}$alkyl, $C_3-C_{18}$alkyl which is interrupted by one or more of O, S or $-NR^6-$, or are $C_5-C_{12}$cycloalkyl, $C_6-C_{14}$aryl, $C_3-C_8$alkenyl, $C_7-C_{15}$aralkyl, $C_6-C_{14}$ aryl or $C_1-C_3$hydroxyalkyl, or $R_4$ and $R_5$, together wit the nitrogen atom, are a pyrrolidine, piperidine, piperazine or morpholine ring.

$R^6$ is H, $C_1-C_{18}$alkyl, $C_5-C_{12}$cycloalkyl, $C_3-C_8$alkenyl, $C_6-C_{14}$aryl or $C_7-C_{18}$aralkyl,

$R^7$ is H, $C_1-C_{18}$alkyl which is unsubstituted or substituted by $-PO(OR^8)_2$, phenyl which is unsubstituted or substituted by OH, or is $C_7-C_{18}$aralkyl or $-CH_2OR^8$,

$R^8$ is $C_1-C_{18}$alkyl, $C_3-C_{18}$alkenyl, $C_5-C_{10}$cycloalkyl, $C_6-C_{16}$aryl or $C_7-C_{15}$aralkyl,

$R^9$ is $C_2-C_8$alkylene, $C_4-C_8$alkenylene, C4alkinylene, cyclohexylene, $C_4-C_{10}$alkylene which is interrupted by one or more $-O-$ atoms, or is

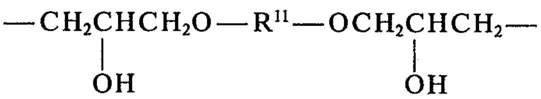 or 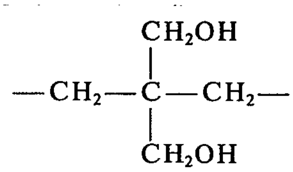

$R^{10}$ is $C_2-C_{12}$alkylene which may be interrupted by one or more $-O-$ atoms or is cyclohexylene,

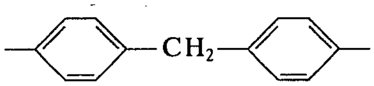 or 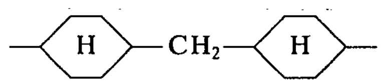

or $R^{10}$ and $R^6$, together with the two nitrogen atoms, are a piperazine ring, and

$R^{11}$ is $C_2-C_8$alkylene, $C_4-C_{10}$alkylene which is interrupted by one or more $-O-$ atoms, or is 1,3- or 1,4-cyclohexylene, 1,3- or 1,4-phenylene,

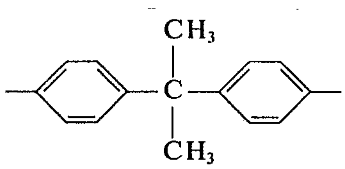 or 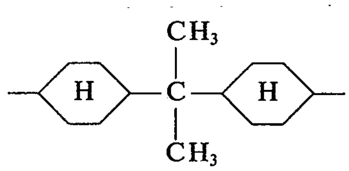

2. A compound of the formula I according to claim 1, wherein $R^1$ is H or Cl, n is 1 and $R^2$ is

$-OR^3$ or 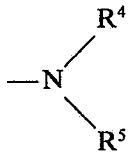

wherein

$R^3$ is $C_4-C_{12}$alkyl, $C_3-C_{10}$alkyl which is interrupted by O, or is cyclohexyl or

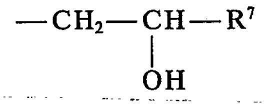

which is unsubstituted or substituted by OH,

$R^4$ and $R^5$ are each independently $C_4-C_{12}$alkyl, $C_3-C_{10}$alkyl which is interrupted by O, or is cyclohexyl,

$R^7$ is $C_1-C_{12}$alkyl which is unsubstituted or substituted by

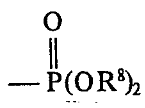

or is $-CH_2OR^8$, and

$R^8$ is $C_1-C_{12}$alkyl or cyclohexyl.

3. A compound of the formula I according to claim 1, wherein $R^1$ is H or Cl, n is 2 and $R^2$ is one of the divalent radicals

$-O-R^9-O-$ or 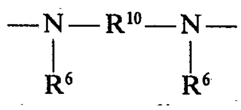

wherein

$R^6$ is H or $C_1-C_8$alkyl,

$R^9$ is $C_2-C_6$alkylene, $C_4-C_{10}$alkylene which is interrupted by $-O-$, or is

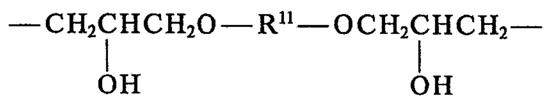

$R^{10}$ is $C_1-C_{12}$alkylene, and

$R^{11}$ is $C_2-C_6$alkylene or is $C_4-C_{10}$alkylene which is interrupted by $-O-$.

4. A compound according to claim 1 selected from

2-[2-hydroxy-3-tert-butyl-5-(2-carbo-n-octyloxyethyl)phenyl]benzotriazole or
2-{2-hydroxy-3-tert-butyl-5-[2-carbo-(2-ethylhexyl)oxyethyl]phenyl}benzotriazole or
2-[2-hydroxy-3-tert-butyl-5-(2-carbo-n-oxtyloxyethyl)-phenyl]-5-chlorobenzotriazole or
2-{2-hydroxy-3-tert-butyl-5-[2-carbo-(2-ethylhexyl)oxyethyl]phenyl}-5-chlorobenzotriazole or

the compound of the formula

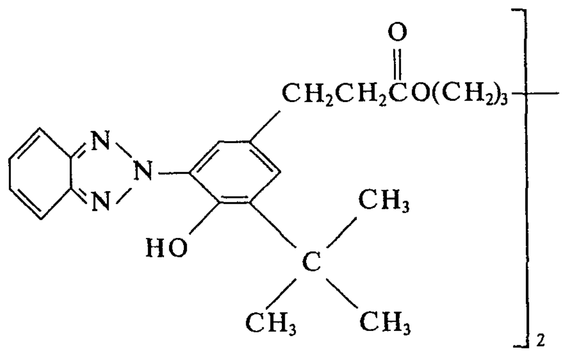

5. A compound according to claim 1, which is a mixture of

2-[2-hydroxy-3-tert-butyl-5-(2-carbo-n-octyloxyethyl)phenyl]-5-chlorobenzotriazole and
2-{2-hydroxy-3-tert-butyl-5-[2-carbo-(2-ethylhexyl)oxyethyl]-phenyl}-5-chlorobenzotriazole

in a ratio by weight of 1 : 1.

6. A stabilised organic material preferably containing 0.1 to 5% by weight of a compound according to claim 1.

7. A stabilised material according to claim 6, which is a synthetic polymer.

8. A stabilised polymer according to claim 7, which is a polyester, polycarbonate, heat-crosslinkable acrylic resin, thermo-plastic acrylic resin, polyamide, MF resin, UF resin or a polyurethane.

9. A stabilised lacquer based on one or more polymers according to claim 8.

10. A stabilised material according to claim 6 which is in the form of photographic material or part of a photographic material, the photographic material containing 0.1 to 5% by weight of a compound of the formula I, preferably in top layers.

11. A process for the preparation of a benzotriazolecarboxylic acid of the formula I according to claim 1, or of an ester or acid chloride thereof, which process comprises coupling a diazotised substituted or unsubstituted o-nitroaniline of the formula II

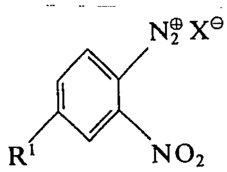

(II)

Wherein $X^{\ominus}$ is a halogen anion, $-HSO_4^{\ominus}$ or $(BF)_4)^{\ominus}$, at pH 8 to 11 with 2-(3-tert-butyl-4-hydroxyphenyl)propionic acid and preparing the corresponding benzotriazolecarboxylic acid from the resultant dye by reductive triazolistion under alkaline conditions, and, if desired, then esterifying the resultant benzotriazole acid, in the presence of an acid catalyst, where n is 1 in formula I, direct with an alcohol of the formula $R^{3\prime}OH$, wherein $R^{3\prime}$ has the same meaning as $R^3$ in formula I but does not mean

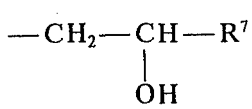

or, where n is 2 in formula I, direct with an alcohol of the formula $HO-R^9$, or reacting the benzotriazole acid with an epoxide compound of the formula

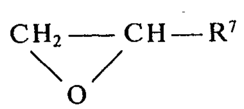

or chlorinating the benzotriazole acid with an excess of thionyl chloride in a hydrocarbon suspension to give the acid chloride.

12. A process for the preparation of a benzotriazolecarboxylic acid ester of the formula I according to claim 1, which process comprises transesterifying a benzotriazolecarboxylic acid ester (preferably the methyl or ethyl ester) which may have been obtaned by the process according to claim 11, in the presence of a transesterification catalyst, such as terabutyl orthotitanate, lithium amide or sodium methylate, where n is 1 in formula I, with an alcohol of the formula $R^{3\prime}OH$, or, where n is 2 in formula I,

with a dialcohol of the formula HO—$R^9$—OH, distilling off the alcohol formed, $R^{3'}$ being as defined in claim 11.

13. A process for the preparation of a benzotriazolecarboxylic acid amide of the formula I according to claim 1, which process comprises reacting, under heat, a benzotriazolecarboxylic acid ester (preferably the methyl or ethyl ester) which may have been obtained by the process according to claim 11, in the presence of a catalyst, such as lithium amide or sodium methylate, where n is 1 in formula I, with ammonia or a primary or secondary amide of the formula

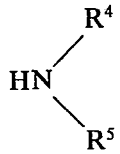

or, where n is 2 in formula I, with a diamine of the formula

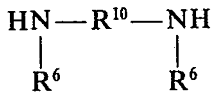

distilling off the alcohol formed.

## Revendications

1. Composés répondant à la formule I:

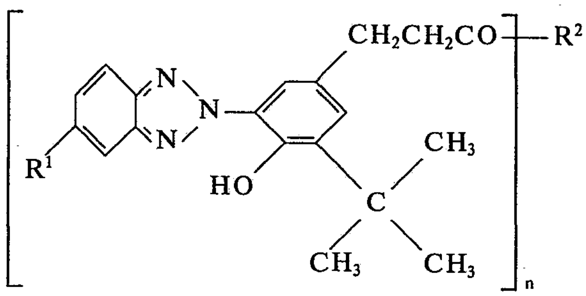

(I)

dans laquelle $R_1$ représente un atome d'hydrogène ou de chlore, un alkyle en $C_1-C_4$ ou un alcoxy en $C_1-C_4$, n représente un nombre égal à 1 ou à 2 et $R_2$ représente:

a) dans le cas où n est égal à 1: un atome de chlore, ou un radical

$—OR^3$ ou 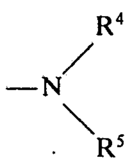

et

b) dans le cas où n est égal à 2: un radical divalent, en l'espèce

$—O—R^9—O—$ ou 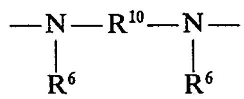

les divers symboles présents dans les radicaux cités ayant les significations suivantes:

$R^3$ représente un atome d'hydrogène, un alkyle en $C_1-C_{18}$ éventuellement porteur d'un à trois radicaux OH ou d'un radical

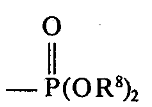 $—O—Si(R^6)_3$ ou $—O—CO(R^6)$

un alkyle en $C_3-C_{18}$ interrompu une ou plusieurs fois par $-O$, $-S$ ou $-NR^6-$, un cycloalkyle en $C_5-C_{12}$ éventuellement porteur d'un radical $-OH$, un alcényle en $C_2-C_{18}$ éventuellement porteur d'un radical $-OH$, un aralkyle en $C_7-C_{15}$, un radical

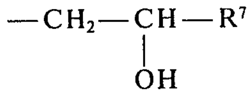 ou un radical 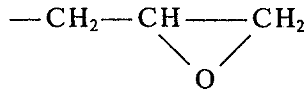

et

$R^4$ et $R^5$ représentent chacun, indépendamment l'un de l'autre, un atome d'hydrogène, un alkyle en $C_1-C_{18}$ un alkyle en $C_3-C_{18}$ interrompu une ou plusieurs fois par $-O-$, $-S-$ ou $-NR^6-$, un cycloalkyle en $C_5-C_{12}$, un aryle en $C_6-C_{14}$, un alcényle en $C_3-C_8$, un aralkyle en $C_7-C_{15}$, un aryle en $C_6-C_{14}$ ou un hydroxyalkyle en $C_1-C_3$, ou encore $R^4$ et $R^5$ forment ensemble, et avec l'atome d'azote, un noyau de pyrrolidine, de pipéridine, de pipérazine ou de morpholine,

$R^6$ représente un atome d'hydrogène, un alkyle en $C_{18}$, un cycloalkyle en $C_5-C_{12}$, un alcényle en $C_3-C_8$ en$C_6-C_{14}$ ou un aralkyle en $C_7-C_{18}$,

$R^7$ représente un atome d'hydrogène, un alkyle en $C_1-C_{18}$ éventuellement porteur d'un radical $-PO(OR^8)_2$, un phényle éventuellement porteur d'un radical $-OH$, un aralkyle en $C_7-C_{18}$ ou un radical $-CH_2OR_8$,

$R^8$ représente un alkyle en $C_1-C_{18}$ un alcényle en $C_3-C_{18}$, un cycloalkyle en $C_5-C_{10}$, un aryle en $C_6-C_{16}$ ou un aralkyle en $C-C_{15}$,

$R^9$ représente un alkylène en $C_2-C_8$, un alcénylène en $C_4-C_8$, un alcynylène en $C_4$, un cyclohexylène, un alkylene en $C_4-C_{10}$ interrompu une ou plusieurs fois par $-O-$,un radical

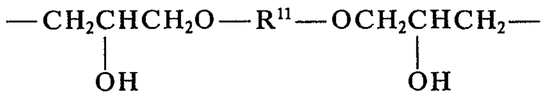 ou un radical 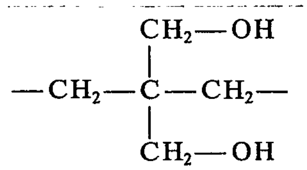

et

$R^{10}$ représente un radical alkylène en $C_2-C_{12}$ éventuellement interrompu une ou plusieurs fois par $-O-$, un cyclohexylène ou un radical de formule:

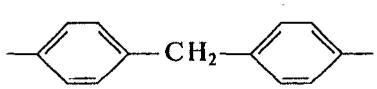 ou 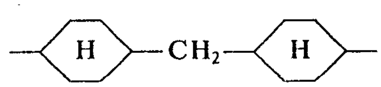

ou encore $R^{10}$ et $R^6$ forment ensemble e avec les deux atomes d'azote un noyau de pipérazine, et

$R^{11}$ représente un alkylène en $C_2-C_8$, un alkylène en $C_4-C_{10}$, interrompu une ou plusieurs fois par $-O-$, un cyclohexylène-1,3 ou -1,4, un phénylène-1,3 ou -1,4, ou un radical de formule:

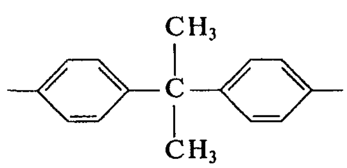 ou 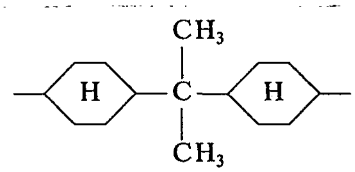

2. Composés de formule I selon la revendication 1 dans lesquels $R^1$ représente H ou Cl, n représente le nombre 1 et $R^2$ représente un radical

$-OR^3$ ou un radical 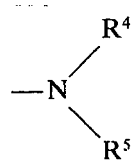

et

$R^3$ représente un alkyle en $C_4-C_{12}$, un alkyle en $C_3-C_{10}$ interrompu par $-O-$, un cyclohexyle ou un radical

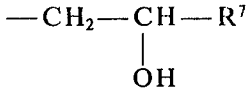

éventuellement porteur d'un —OH,
$R^4$ et $R^5$ représentent chacun, indépendamment l'un de l'autre, un alkyle en $C_4-C_{12}$, un alkyle en $C_3-C_{10}$ interrompu par —O— ou un cyclohexyle,
$R^7$ représente un alkyle en $C_1-C_{12}$ éventuellement porteur d'un radical

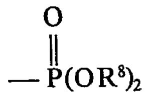

ou un radical $-CH_2OR^8$, et
$R^8$ représente un alkyle en $C_1-C_{12}$ ou un cyclohexyle.

3. Composés de formule I selon la revendication 1 dans lesquels; $R^1$ représente H ou Cl, n représente le nombre 2, $R^2$ représente un radical divalent répondant à l'une des formules

$-O-R^9-O-$ et 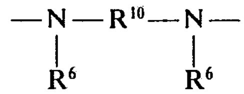

dans lesquelles:

$R^6$ représente H ou un alkyle en $C_1-C_8$,
$R^9$ représente un alkylène en $C_2-C_6$, un alkylène en $C_4-C_{10}$ interrompu par —O— ou un radical

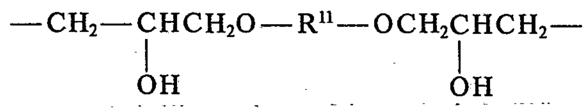

$R^{10}$ représente un alkylène en $C_2-C_{12}$ et
$R^{11}$ représente un alkylène en $C_2-C_6$ ou un alkylène en $C_4-C_{10}$ interrompu par —O—.

4. Composé selon la revendication 1, pris dans l'ensemble constitué par:

1'[hydroxy-2 tert-butyl-3 (n-octyloxycarbonyl-2 éthyl)-5 phényl]-2 benzotriazole,
1'{hydroxy-2 tert-butyl-3 [(éthyl-2 hexyloxycarbonyl)-2 éthyl]-5 phényl}-2 benzotriazole,
1'[hydroxy-2 tert-butyl-3 (n octyloxycarbonyl-2 éthyl)-5 phényl]-2 chloro-5 benzotriazole,
1'{hydroxy-2 tert-butyl-3 [(éthyl-2 hexyloxycarbonyl)-2 éthyl]-5 phényl}-2 chloro-5 benzotriazole et le composé de formule:

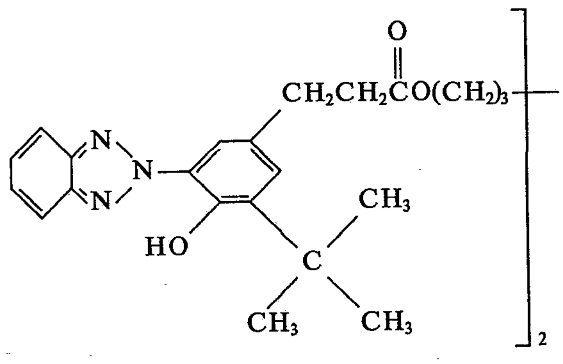

5. Composé selon la revendication 1, caractérisé en ce qu'il s'agit d'un mélange, dans un rapport pondéral de 1 : 1,

d'[hydroxy-2 tert-butyl-3 (n-octyloxycarbonyl-2 éthyl)-5 phényl]-2 chloro-5 benzotriazole et
d'{hydroxy-2 tert-butyl-3 [(éthyl-2 hexyloxycarbonyl)-2 éthyl]-5 phényl}-2 chloro-5 benzotriazole.

6. Matière organique stabilisée qui contient, dans sa mass, de préference de 0,1 à 5% en poids d'un composé selon la revendication 1.
7. Matière stabilisée selon la revendication 6, caractérisée en ce que ladite matière est un polymère synthétique.
8. Polymère stabilisé selon la revendication 7, caractérisée en ce que ce polymère est un polyester, un polycarbonate, une résine acrylique réticulable à chaud, une résine acrylique thermoplastique, un polyamide, une résine MF ou UF, ou un polyuréthanne.
9. Peintures et vernis stabilisés à base d'un ou plusieurs polymères selon la revendication 8.

10. Matière stabilisée selon la revendication 6, caractérisée en ce qu'il s'agit d'un produit photographique ou d'une partie d'un produit photographique, le produit photographique, qui est de préférence sous la forme de couches de recouvrement, contenant de 0,1 à 5% en poids d'un composé de formule I.

11. Procédé de préparation de composés de formule I selon la revendication 1, plus précisément d'acides benzotriazole carboxyliques, de leurs esters et de leurs chlorures d'acides, procédé selon lequel on copule une o-nitraniline diazotée, éventuellement substituée, répondant à la formule II:

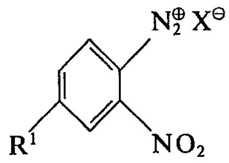

(II)

dans laquelle $X^{\ominus}$ représente un anion halogène, $-HSO_4^{\ominus}$ ou $(BF_4)^{\ominus}$, avec l'acide (tert-butyl-3 hydroxy-4 phényl)-2 propionique, à un pH 8 à 11, et, à partir du colorant obtenu, on prépare, par triazolation réductrice, dans des conditions alcalines, l'acide benzotriazole-carboxylique correspondant.
et ensuite, le cas échéant, on estérifie directement l'acide benzotriazole-carboxylique obtenu, en présence d'un catalyseur acide, soit, dans le cas où n est égal 1 dans la formule I, avec un alcool $R^{3'}OH$ dans lequel $R^{3'}$ a la signification qui a été donnée pour $R^3$ à propos de la formule I sans toutefois pouvoir représenter un radical

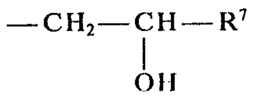

soit, dans le cas où n est égal à 2 dans la formule I, avec un alcool $HO-R^9-OH$,
ou on fait réagir l'acide benzotriazole-carboxylique avec un composé époxydique de formule:

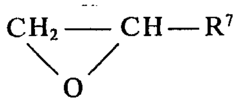

ou on clore l'acide benzotriazole-carboxylique au moyen d'un excès de chlorure de thionyle, en suspension dans un hydrocarbure, de manière à le convertir en chlorure d'acide.

12. Procédé de préparation d'esters d'acides benzotriazole-carboxyliques de formule I selon la revendication 1, procédé caractérisé en ce qu'on transestérifie un ester d'acide benzotriazole-carboxylique (de préférence un ester méthylique ou éthylique), obtenu éventuellement par le procédé de la revendication 11, en présence de catalyseurs de transestérification, tels que l'orthotitanate de tétrabutyle, l'amidure de lithium ou le méthylate de sodium, avec des alcools $R^{3'}OH$ dans le cas où n est égal à 1 dans la formule I ou avec des diols $HO-R^9-OH$ dans le cas où n est égal à 2 dans la formule I, tout en chassant par distillation l'alcool libéré (le symbole $R^{3'}$ a la signification qui lui a été donné à la revendication 11).

13. Procédé de préparation d'amides d'acides benzotriazole-carboxyliques de formule I selon la revendication 1, procédé caractérisé en ce qu'on fait réagir à chaud un ester d'acide benzotriazole-carboxylique (de préférence un ester méthylique ou éthylique), éventuellement obtenu par le procédé de la revendication 11, en présence de catalyseurs, tels que l'amidure de lithium ou le méthylate de sodium, avec, dans le cas où n est égal à 1 dans la formule I, l'ammoniac ou une amine primaire ou secondaire de formule:

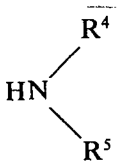

ou, dans le cas où n est égal à 2 dans la formule I, une diamine de formule:

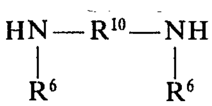

à chaque fois en chassant par distillation l'alcool libéré.